# EUROPEAN PATENT APPLICATION

(11) **EP 4 553 069 A1**
(43) Date of publication of application: **14.05.2025**
(21) Application number: 23834932.8
(22) Date of filing: 06.07.2023
(51) Int. Cl.: C07D 263/26, C07D 277/14, C07D 277/18, C07D 405/10, C07D 409/06, C07D 417/10, A61K 31/422, A61K 31/426, A61P 25/28

(54) **ALPHA,ß-UNSATURATED AMIDE COMPOUND, AND PREPARATION METHOD THEREFOR, AND PHARMACEUTICAL COMPOSITION AND USE THEREOF**

(30) Priority: 06.07.2022 CN 202210800410
(71) Applicant: Shanghai Institute of Materia Medica, Chinese Academy of Sciences, Shanghai 201203 (CN); Kunming Institute of Zoology, Chinese Academy of Sciences, Yunnan 650223 (CN)
(72) Inventor: LIU, Hong, Shanghai 201203 (CN); XU, Lin, Kunming, Yunnan 650223 (CN); ZHOU, Yu, Shanghai 201203 (CN); TAN, Yahong, Kunming, Yunnan 650223 (CN); WANG, Yibing, Shanghai 201203 (CN); LI, Jinnan, Kunming, Yunnan 650223 (CN); HUANG, He, Shanghai 201203 (CN); ZHOU, Qixin, Kunming, Yunnan 650223 (CN); CHENG, Yilang, Shanghai 201203 (CN)
(74) Representative: dompatent von Kreisler Selting Werner - Partnerschaft von Patent- und Rechtsanwälten mbB
(86) International application number: PCT/CN2023/106181
(87) International publication number: WO 2024/008166

(57) **Abstract**

Provided in the present invention are an α,β-unsaturated amide compound, and a preparation method therefor, and a pharmaceutical composition and the use thereof. Specifically, provided in the present invention is a compound as represented by formula I, wherein the definition of each group is as described in the description. The compound can be used as a compound for improving cerebral blood flow and is used for preparing a pharmaceutical composition for treating neurodegenerative diseases such as Alzheimer's disease and vascular dementia and strokes.

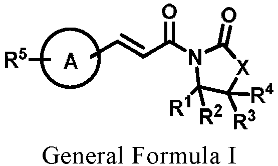

## Description

### Technical field

The present invention relates to the field of medicinal chemistry and medicine, specifically to a class of an α,β-unsaturated amide compound, preparation method therefor, pharmaceutical composition containing the same, and their use as drugs for treating neurodegenerative diseases such as Alzheimer's disease, vascular dementia, and stroke.

### Background

Alzheimer's disease (AD) is a degenerative brain disease that accounts for about twothirds of dementia in the elderly. Its main symptoms include progressive memory loss, β - amyloid deposition (A β), and neurofibrillary tangles (NFT). The pathological mechanism of AD is very complex, and existing marketed drugs can only temporarily alleviate or control AD symptoms. Effective pathways for targeted disease mechanism therapy have not yet been discovered. Aging is the main risk factor for Alzheimer's disease, with over 95% of AD patients having late-onset onset, occurring at the age of 65 or older. Less than 5% of AD is early-onset, and the main cause of the disease is genetic factors. Cardiovascular and cerebrovascular diseases were also major risk factors for AD, and vascular changes may contribute to the pathological development of AD, not just vascular dementia caused by known vascular lesions. As the most abundant and smallest vascular unit in the brain, capillaries induce damage to cerebral blood flow (CBF) or blood-brain barrier (BBB), which is associated with memory decline in AD.

More and more studies have found that in the early stages of AD and normal aging mild cognitive impairment (MCI), there is a decrease in cerebral blood flow, reactive damage to cerebral blood vessels, and impaired hemodynamic responses. Early research has found that transcranial Doppler measurements of the middle cerebral artery indicate that individuals with higher cerebral blood flow velocity were less likely to develop dementia or atrophy of the hippocampus and amygdala. Arterial spinlabeling MRI shows reduced cerebral blood flow in the posterior dentate gyrus and anterior process in patients with early MCI or AD. In elderly people at high risk of AD, decreased or disrupted cerebral blood flow occurs before cognitive decline, brain atrophy, and accumulation of beta amyloid protein. APOE is the main genetic risk factor for AD and also a susceptibility gene for vascular disease. In animal models, reduced cerebral blood flow and vascular dysfunction were also observed in transgenic mice targeting human APOE4 gene 125-127 instead of mouse apolipoprotein E4 (APOE). It was also found that the vascular phenotype of mice expressing APOE4 preceded neuronal and synaptic dysfunction. Insufficient perfusion can induce or exacerbate Alzheimer's disease like neuronal dysfunction and neuropathological changes. A 50% reduction in chronic blood flow will lead to significant cognitive changes, a sustained decrease of more than 20% in cerebral blood flow will result in loss of attention, and a decrease of more than 30% in rat cerebral blood flow will impair spatial memory. A decrease in cerebral blood flow reduces the activity of Na+/K+pumps and all processes dependent on them, including maintaining resting potential and glutamate uptake. It also leads to the production of adenosine, which inhibits glutamate release and affects neuronal function. Bilateral carotid artery occlusion in rats can lead to memory impairment, neuronal dysfunction, synaptic changes, and accumulation of neurotoxic beta amyloid oligomers. Cerebral ischemia, hypoxia, and A β deposition were interdependent. Insufficient perfusion can trigger accelerated deposition of A β. On the contrary, A β can damage cerebral vascular function, increase arterial constriction, and reduce cerebral blood flow. In rodents, ischemia leads to the accumulation and formation of highly phosphorylated Tau in neurons, similar to the filaments in human neurodegenerative diseases and Alzheimer's disease. Insufficient perfusion affects the structural and functional changes of the brain and provides promising potential biomarkers for identifying and diagnosing preclinical Alzheimer's disease.

Capillaries were the smallest blood vessels in the brain, branching off from small arteries to form a rich network of microvessels. The maximum surface werea of capillaries per gram of brain is approximately 120 cm2. The capillary network is mainly composed of endothelial cells, basement membrane, pericytes, and astrocytes. Capillary dysfunction has been observed in animal models as a precursor to neurodegenerative changes associated with dementia. The constriction of capillaries in AD leads to hypoxia in nerve tissue, which may also be the reason for the decrease in glucose metabolism in AD. Moreover, ischemia and hypoxia have been shown to upregulate the enzyme β - secretase (BACE1) responsible for generating A β. Capillary blood flow is mainly regulated by pericytes, and extensive research has found that pericyte damage is closely related to AD. In some brain anatomical samples of AD patients, the level of pericytes can even decrease by as much as 50%. In transgenic mice with defects in pericytes, blood flow decreases, microvessels decrease, A β expression increases, and blood-brain barrier permeability increases. Many neuropathological studies have described morphological changes in cerebral capillaries and periventricular white matter lesions similar to ischemic infarction in AD. Autopsy studies of late stage Alzheimer's patients have shown that a large number of capillary endothelial cells in the brain have shed, the blood vessel walls have collapsed, and the density has decreased. The fine and unique structure of capillaries increases their susceptibility to damage, and capillary degeneration is more frequent and common than vascular amyloidosis, potentially indicating that the long-term degradation process of the AD brain microvascular system may be partially independent of amyloid toxicity. More and more studies have shown that vascular factors were the main risk factors for AD, and damage to neurovascular units is associated with AD. Therefore, seeking interventions to increase cerebral blood flow through capillaries may be of great significance for the prevention and treatment of AD.

The present invention provides a target compound that targets capillaries to increase cerebral blood flow and improve AD symptoms, which is used for the treatment of neurodegenerative diseases such as Alzheimer's disease, vascular dementia, and stroke in clinical practice.

### Summary

One purpose of the present invention is to provide an α,β unsaturated amide compound represented by formula I, or a pharmaceutically acceptable salt, a racemate, a R-isomer, a S-isomer, or a mixture thereof.

Another purpose of the present invention is to provide a method for preparing the α,β unsaturated amide compound represented by the above formula I.

Another purpose of the present invention is to provide a method for treating neurodegenerative diseases such as Alzheimer's disease, vascular dementia, and stroke related to cerebral blood flow, comprising administering to a patient in need of such treatment one or more of the α,β unsaturated amide compounds selected from the above formula I, a pharmaceutically acceptable salt, a racemate, a R-isomers, a S-isomers, or a mixture thereof.

A first aspect of the present invention provides an α,β-unsaturated amide compound of formula I, or a racemic, a R-isomer, a S-isomer, a pharmaceutically acceptable salt, or a mixture thereof: 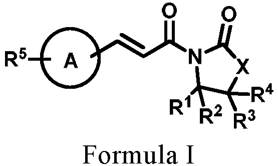 wherein,
R¹, R², R³, and R⁴ can each be independently selected from the group consisting of hydrogen, deuterium, tritium, halogen, cyano, amino, hydroxyl, nitro, substituted or unsubstituted C1~C6 alkyl, substituted or unsubstituted C1~C6 alkoxy, or (CHR⁶)ₙR;wherein, R is selected from the group consisting of: substituted or unsubstituted C6-C10 aryl, substituted or unsubstituted 5-7 membered heteroaryl, substituted or unsubstituted 5-7 membered heterocyclyl, substituted or unsubstituted C3-C12cycloalkyl or fused heterocycle; or R³ and R⁴ together with the attached carbon atom form a group selected from the group consisting of carbonyl, substituted or unsubstituted 3-8-membered cycloalkyl, or substituted or unsubstituted 4-8 membered heterocyclyl;
^{Ⓐ}ring is selected from the group consisting of: C6-C10 aryl, 5-12 membered heteroaryl, 5-12 membered heterocyclyl, C3-C12cycloalkyl, or fused heterocycle;
R⁵ is 1, 2, 3, 4 or 5 substituents located on ^{Ⓐ} ring selected from the group consisting of hydrogen, deuterium, tritium, halogen, cyano, amino, hydroxyl, nitro, substituted or unsubstituted C1-C6 alkyl, substituted or unsubstituted C1-C6 alkoxy, substituted or unsubstituted C6-C10 aryl, substituted or unsubstituted C6-C10 aryloxy, substituted or unsubstituted 5-7 membered heteroaryl, substituted or unsubstituted 5-7 membered heteroaryloxy, substituted or unsubstituted 5-7 membered heterocycle, substituted or unsubstituted C3-C12 cycloalkyl;
or two adjacent R⁵ are connected end-to-end with the atoms on Ⓐ ring to form a substituted or unsubstituted 4-8 membered ring (i.e., forming a fused ring structure with the A ring);
or two R⁵ on the same atom of Ⓐ ring are connected end-to-end together withⒶ ring form a substituted or unsubstituted 3-8-membered ring (i.e., forming a spiro ring structure with ring A);
X is N (CH₂) ₙR⁶, O, or S;
n is 0, 1, 2, or 3;
R⁶ is independently selected from the group consisting of: hydrogen, halogen, cyano, amino, hydroxyl, nitro, substituted or unsubstituted C1-C6 alkyl, substituted or unsubstituted C1-C6 alkoxy, substituted or unsubstituted C6-C10 aryl, substituted or unsubstituted 5-12 membered heterocyclyl containing 1 to 3 heteroatoms selected from oxygen, sulfur, and nitrogen, substituted or unsubstituted C2-C10 acyl, substituted or unsubstituted C2-C10 ester group, substituted or unsubstituted C1-C6 amide group, -SO₂R⁵, and -COR⁵;
wherein, unless otherwise specified, the heteroaromatic ring, heterocycle, or heterocyclyl are each independently contains 1 to 4 heteroatoms selected from oxygen, sulfur, and nitrogen; the aromatic or heteroaromatic ring includes a monocyclic, fused-ring, or fused ring, and the carbocycle or heterocycle includes a monocyclic, fused, spiro, or bridged ring;
the "substituted" refers to being substituted by one or more (preferably 1-3) substituents selected from the group consisting of halogen, cyano, nitro, amino, hydroxyl, hydroxymethyl, carboxyl, thiol, C1-C6 alkyl, halogen substituted C1-C6 alkyl, C1-C6 alkoxy, halogen substituted C1-C6 alkoxy, C1-C6 alkoxycarbonyl, C2-C6 alkenyl, C2-C6 alkynyl, C3-C8 cycloalkyl, C1-C6 alkylsulfonyl, substituted or unsubstituted C6-C10 aryl, substituted or unsubstituted 5-7 membered heteroaryl, and 3-12 membered heterocyclyl;
the halogen is F, Cl, Br or I.

In another preferred embodiment, Ⓐ ring is selected from the group consisting of C6~C10 aryl and C5~C12 heteroaryl.

In another preferred embodiment, R¹, R², R³ and R⁴ are each independently selected from the group consisting of hydrogen, deuterium, substituted or unsubstituted C1-C6 alkyl, or (CHR⁶)ₙR; wherein R is selected from the group consisting of: substituted or unsubstituted C6-C10 aryl and substituted or unsubstituted 5-7 membered heteroaryl; n is 0, 1 or 2; R⁶ is hydrogen, halogen, or substituted or unsubstituted C1-C6 alkyl.

In another preferred embodiment, R¹, R², R³, and R⁴ are each independently selected from the group consisting of hydrogen, deuterium, substituted or unsubstituted C1-C6 alkyl and (CHR⁶)ₙR; wherein R is selected from the group consisting of: substituted or unsubstituted C6-C10 aryl and substituted or unsubstituted 5-7 membered heteroaryl.

In another preferred embodiment, R³ and R⁴ are each independently selected from the group consisting of hydrogen, deuterium, and substituted or unsubstituted C1-C6 alkyl.

In another preferred embodiment, R¹ is H or D, and R² is selected from the group consisting of hydrogen, deuterium, substituted or unsubstituted C1-C6 alkyl and (CHR⁶)nR; wherein R is selected from the group consisting of: substituted or unsubstituted C6-C10 aryl, substituted or unsubstituted 5-7 membered heteroaryl; the "substituted" refers to being substituted by one or more substituents selected from the group consisting of halogen, cyano, nitro, amino, hydroxyl, hydroxymethyl, carboxyl, thiol, C1-C6 alkyl, halogen substituted C1-C6 alkyl, C1-C6 alkoxy, halogen substituted C1-C6 alkoxy, and C1-C6 alkoxycarbonyl.

In another preferred embodiment, R³ and R⁴ are each independently deuterium.

In another preferred embodiment, R¹ and R² are each independently deuterium.

In another preferred embodiment, the compound of formula I is the compound described in each Example.

A second aspect of the present invention provides a method for preparing the compound of formula I as described in the first aspect of the present invention, comprising steps of:

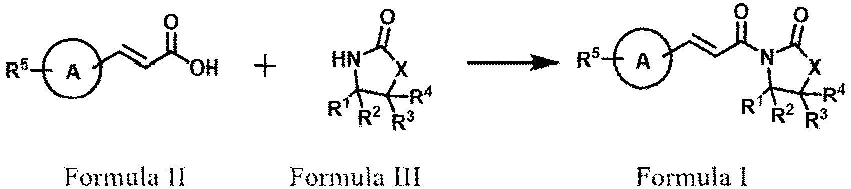

In an inert solvent, reacting a compound of formula II and a compound of formula III to obtain the compound of formula I.

In another preferred embodiment, the reaction is carried out in the presence of an organic amine and pivaloyl chloride.

In another preferred embodiment, the organic amine is triethylamine.

A third aspect of the present invention provides a pharmaceutical composition comprising (1) a compound as described in the first aspect of the present invention, or a stereoisomer or tautomer , a pharmaceutically acceptable salt, a hydrate or a solvate thereof; and (2) a pharmaceutically acceptable carrier.

A fourth aspect of the present invention provides a use of the compound as described in the first aspect of the present invention, a stereoisomer, a tautomer, a pharmaceutically acceptable salt, a hydrate or solvate thereof, or a pharmaceutical composition of claim 9 in the preparation of a pharmaceutical composition for the prevention and/or treatment of neurodegenerative diseases or stroke.

In another preferred embodiment, the neurodegenerative disease is selected from the group consisting of Alzheimer's disease and vascular dementia.

It should be understood that within the scope of the present invention, the above-mentioned technical features of the present invention and the specific technical features described in the following examples can be combined with each other to form new or preferred technical solutions. Due to space limitations, it will not repeat here.

### Figures

Figure 1. The flowchart of the laser speckle test blood flow model in pharmacological activity experiment Example 1;
Figure 2. The blood flow improvement effect of the compound of the present invention.

### Examples

After long-term and in-depth research, the inventor has provided an α,β unsaturated amide compound that can be used for neurodegenerative diseases such as Alzheimer's disease. The compound can effectively improve capillary cerebral blood flow. Based on the above findings, the inventor has completed the present invention.

### Term

In the present invention, the halogen is F, Cl, Br, or I.

In the present invention, unless otherwise specified, the terms used herein have general meanings known to those skilled in the art.

In the present invention, the term "C1-C6 alkyl" refers to straight or branched alkyl having 1 to 6 carbon atoms, including but not limited to methyl, ethyl, propyl, isopropyl, butyl, isobutyl, sec butyl, tert butyl, pentyl, and hexyl, etc; Preferred is ethyl, propyl, isopropyl, butyl, isobutyl, sec butyl, and tert butyl.

In the present invention, the term "C1-C6 alkoxy" refers to straight or branched chain alkoxy having 1 to 6 carbon atoms, including but not limited to methoxy, ethoxy, propoxy, isopropoxy, and butoxy.

In the present invention, the term "C2-C6 alkenyl" refers to a straight or branched chain alkenyl containing one double bond with 2 to 6 carbon atoms, including but not limited to vinyl, propenyl, butenyl, isobutenyl, pentenyl, and hexenyl.

In the present invention, the term "C2-C6 alkynyl" refers to a straight or branched chain alkynyl containing a triple bond with 2 to 6 carbon atoms, including but not limited to ethynyl, propynyl, butynyl, isobutyl, pentynyl, and hexynyl.

In the present invention, the term "C3-C10 cycloalkyl" refers to a cycloalkyl having 3 to 10 carbon atoms on the ring, including but not limited to cyclopropyl, cyclobutyl, cyclopentyl, cyclohexyl, cycloheptyl, cyclooctyl, and cyclodecyl. The terms" C3-C8 cycloalkyl ", " C3-C7 cycloalkyl ", and" C3-C6 cycloalkyl "have similar meanings.

In the present invention, the term "C3-C10 cycloalkenyl" refers to a cycloalkenyl having 3 to 10 carbon atoms on the ring, including but not limited to cyclopropenyl, cyclobutenyl, cyclopentenyl, cyclohexenyl, cycloheptenyl, cyclooctenyl, and cyclodecyl. The term "C3-C7 cycloalkenyl" has a similar meaning.

In the present invention, the terms "aromatic ring" or "aryl" have the same meaning, and preferably "aryl" is "C6-C12 aryl" or "C6-C10 aryl". The term 'C6-C12 aryl' refers to aromatic ring groups with 6 to 12 carbon atoms that do not contain heteroatoms on the ring, such as phenyl, naphthyl, etc. The term 'C6-C10 aryl' has a similar meaning.

In the present invention, the terms "aromatic heterocycle" or "heteroaryl" have the same meaning, referring to a heteroaromatic group containing one or more heteroatoms. The heteroatoms referred to here include oxygen, sulfur, and nitrogen. For example, furyl, thienyl, pyridyl, pyrazolyl pyrazole, pyrrolyl, N-alkylpyrrolyl, pyrimidinyl, pyrazinyl, imidazolyl, tetrazolyl, etc. The heteroaromatic ring can be fused onto an aryl, heterocyclyl, or cycloalkyl ring, wherein the ring connected to the parent structure is a heteroaromatic ring. Heteroaryl can be optionally substituted or unsubstituted.

In the present invention, the term "3-12 membered heterocyclyl" refers to a saturated or unsaturated 3-12 membered ring group containing 1-3 heteroatoms selected from oxygen, sulfur, and nitrogen on the ring, such as dioxolane. The term "3-7 membered heterocyclyl" has a similar meaning.

In the present invention, the term 'substituted' refers to one or more hydrogen atoms on a specific functional group are substituted by a specific substituent. The specific substituents were those described in the preceding text or those that appear in various Examples. Unless otherwise specified, a substituted group may have a substituent selected from a specific group at any substitutable site of the group, which may be the same or different at each position. A cyclic substituent, such as a heterocycloalkyl, can be attached to another ring, such as a cycloalkyl, to form a spirobicyclic system, for example, where two rings share a common carbon atom. Those skilled in this field should understand that the expected combinations of substituents in the present invention were those that were stable or chemically achievable. The substituents include (but are not limited to): C1-8 alkyl, C2-8 alkenyl, C2-8 alkynyl, C3-8 cycloalkyl, 3- to 12 membered heterocyclyl, aryl, heteroaryl, halogen, hydroxyl, carboxyl (- COOH), C1-8 aldehyde, C2-10 acyl, C2-10 ester group, amino, alkoxy, C1-10 sulfonyl, etc.

### Compound of formula I

The present invention provides an α,β-unsaturated amide compound of Formula I, or a racemic, a R-isomer, a S-isomer, a pharmaceutically acceptable salt, or a mixture thereof: 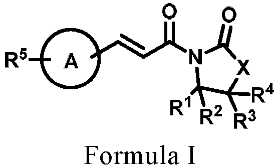 wherein,
R¹, R², R³, and R⁴ can each be independently selected from the group consisting of hydrogen, deuterium, tritium, halogen, cyano, amino, hydroxyl, nitro, substituted or unsubstituted C1~C6 alkyl, substituted or unsubstituted C1~C6 alkoxy, and (CHR⁶)ₙR; wherein R is selected from the group consisting of: substituted or unsubstituted C6-C10 aryl, substituted or unsubstituted 5-7 membered heteroaryl, substituted or unsubstituted 5-7 membered heterocyclyl, substituted or unsubstituted C3-C12cycloalkyl or fuesd heterocycle;
Ⓐring is selected from the group consisting of: C6-C10 aryl, 5-12 membered heteroaryl, 5-12 membered heterocyclyl, C3-C12cycloalkyl or heterocycle;
R⁵ is 1, 2, 3, 4 or 5 substituents located on Ⓐ ring selected from the group consisting of hydrogen, deuterium, tritium, halogen, cyano, amino, hydroxyl, nitro, substituted or unsubstituted C1-C6 alkyl, substituted or unsubstituted C1-C6 alkoxy, substituted or unsubstituted C6-C10 aryl, substituted or unsubstituted C6-C10 aryloxy, substituted or unsubstituted 5-7 membered heteroaryl, substituted or unsubstituted 5-7 membered heteroaryloxy, substituted or unsubstituted 5-7 membered heterocycle, substituted or unsubstituted C3-C12 cycloalkyl;
or two adjacent R⁵ are connected end-to-end with the atoms on 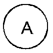 ringform a substituted or unsubstituted 4-8 membered ring (i.e., forming a fused ring structure with the A ring);
or two R⁵ on the same atom on 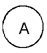 ring are connected end-to-end together with 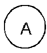 form a substituted or unsubstituted 3-8-membered ring (i.e., forming a spiro ring structure with ring A);
X is N(CH₂)ₙR⁶, O, or S;
n is 0, 1, 2, or 3;
R⁶ is independently selected from the group consisting of: hydrogen, halogen, cyano, amino, hydroxyl, nitro, substituted or unsubstituted C1-C6 alkyl, substituted or unsubstituted C1-C6 alkoxy, substituted or unsubstituted C6-C10 aryl, substituted or unsubstituted 5-12 membered heterocycle containing 1 to 3 heteroatoms selected from oxygen, sulfur, and nitrogen, substituted or unsubstituted C2-C10 acyl, substituted or unsubstituted C2-C10 ester group, substituted or unsubstituted C1-C6 amide group, SO₂R⁵ and -COR⁵;
wherein, unless otherwise specified, the heteroaromatic ring, heterocycle ring, or heterocyclyl are each independently contains 1 to 4 heteroatoms selected from oxygen, sulfur, and nitrogen; the aromatic or heteroaromatic ring includes a monocyclic, fused-ring, or fused ring, and the carbocycle or heterocycle includes a monocyclic, fused, spiro, or bridged ring;
the "substituted" refers to being substituted by one or more (preferably 1-3) substituents selected from the group consisting of halogen, cyano, nitro, amino, hydroxyl, hydroxymethyl, carboxyl, thiol, C1-C6 alkyl, halogen substituted C1-C6 alkyl, C1-C6 alkoxy, halogen substituted C1-C6 alkoxy, C1-C6 alkoxycarbonyl, C2-C6 alkenyl, C2-C6 alkynyl, C3-C8 cycloalkyl, C1-C6 alkylsulfonyl, substituted or unsubstituted C6-C10 aryl, substituted or unsubstituted 5-7 membered heteroaryl, and 3-12 membered heterocyclyl;
the halogen is F, Cl, Br or I.

In a preferred embodiment, R¹, R², R³, R⁴, R⁵, R⁶, Ⓐ ring, X, Ⓐ, S, or n are each independently the corresponding group in the compound in each example.

### Preparation method for compound of formula I

The present invention also provides a method for preparing a compound represented by formula I, which is carried out according to the following scheme (example):
The compound of formula (I) can be prepared by the method shown in Scheme 1 below

The structural formulas and R group labels used in the following schemes were only used in this section. The compounds of formula (II) and formula (III) can be obtained on the market or synthesized using conventional techniques in this field.

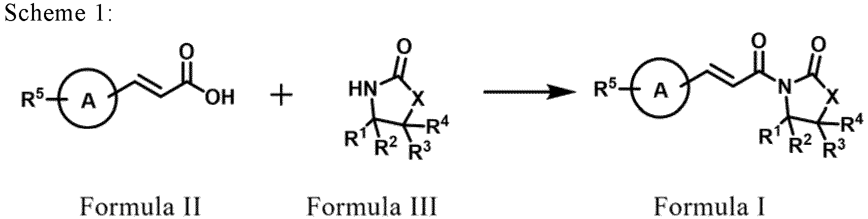

### Pharmaceutical composition

Another aspect of the present invention provides a pharmaceutical composition comprising a therapeutic effective amount selected from one or more of a compound of Formula I, a pharmaceutically acceptable salt, a enantiomer, a diastereomer, and a racemate, and optionally, one or more pharmaceutically acceptable carriers, excipients, adjuvants, accessories and/or diluents. The accessories can be, such as odorants, fragrances, sweeteners, etc.

The pharmaceutical composition provided by the present invention preferably contains active ingredients in a weight ratio of 1-99%. The preferred ratio is that the compound of formula I as the active ingredient accounts for 65wt% to 99wt% of the total weight, and the remaining part is a pharmaceutically acceptable carrier, diluent, solution or salt solution.

The compound and pharmaceutical composition provided by the present invention can be various forms, such as tablets, capsules, powders, syrups, solutions, suspensions, and aerosols, and can be present in suitable solid or liquid carriers or diluents and in suitable disinfectants for injection or drip.

The various dosage forms of the pharmaceutical composition of the present invention can be prepared according to conventional preparation methods in the pharmaceutical field. A unit measurement of its formulation includes 1 mg-700 mg of compound of formula I, and preferably, the unit measurement of the formulation includes 25 mg-300 mg of compound of formula I.

The compounds and pharmaceutical compositions of the present invention can be used clinically in mammals, including humans and animals, and can be administered through oral, nasal, dermal, pulmonary, or gastrointestinal routes. The most preferred option is oral administration. The most preferred daily dose is 50-1400 mg/kg body weight, taken in one dose, or 25-700 mg/kg body weight in divided doses. Regardless of the method of administration, the optimal dosage for an individual should be determined based on the specific treatment. Usually, it starts from a small dose and gradually increases until the most suitable dose is found.

Another aspect of the present invention provides a method for increasing cerebral blood flow, comprising selected from one or more of a compound of Formula I, a pharmaceutically acceptable salt, a enantiomer, a diastereomer and a racemate, and optionally, one or more pharmaceutically acceptable carriers, excipients, adjuvants, accessories and/or diluents.

The compounds and compositions of the present invention were used for the treatment and prevention of neurodegenerative diseases and stroke related to cerebral blood flow, including but not limited to Alzheimer's disease, vascular dementia, and stroke.

The present invention will be further explained in conjunction with specific examples. It should be understood that these examples were only used to illustrate the present invention and not to limit the scope of the present invention. The experimental methods without specific conditions specified in the following examples were usually carried out under conventional conditions or conditions recommended by the manufacturer. Unless otherwise specified, percentages and portions are calculated by weight.

### Example 1 (E) -3- (3- (o-methylphenyl) acryloyl) oxazolidin-2-one

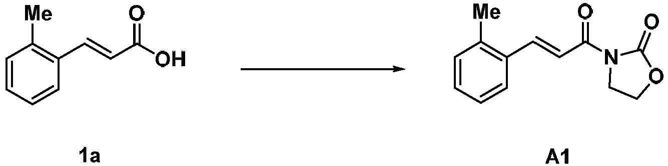

To a solution of compound (E) -3- (o-methylphenyl) acrylic acid 1a (400.0 mg, 2.47 mmol) and triethylamine (685.6 µ L, 4.93 mmol) in ultra dry dichloromethanewas added pivaloyl chloride (364.5 µ L, 2.96 mmol) at -78 °C and stirred at room temperature for 1 hour under argon protection. Then oxazolidin-2-one (214.8 mg, 2.47 mmol) and lithium chloride (104.5 mg, 2.47 mmol) was added at -78 °C and stirred at room temperature for 12 hours. After TLC monitored the completion of the reaction, it was quenched with water and extracted with dichloromethane. The organic layers were combined and washed with saturated sodium chloride solution. Then the organic layer was dried with anhydrous sodium sulfate and concentrated, the crude product was subjected to column chromatography to obtainthe target product (E) -3- (3- (o-methylphenyl) acryloyl) oxazolidin-2-one (490 mg, white solid), yield 85.9%. ¹H NMR (400 MHz, DMSO-d₆) δ 7.96 (d, J = 15.7 Hz, 1H), 7.73 (d, J = 15.7 Hz, 1H), 7.61 (d, J = 8.7 Hz, 1H), 7.38 - 7.31 (m, 1H), 7.30 (d, J = 3.9 Hz, 2H), 4.47 - 4.37 (m, 2H), 4.06 - 3.97 (m, 2H), 2.41 (s, 3H). LRMS (ESI): 232.09 [M+H]+.

### Example 2 (E) -3- (3- (m-methylphenyl) acryloyl) oxazolidin-2-one

Replace (E) -3- (o-methylphenyl) acrylic acid with (E) -3- (m-methylphenyl) acrylic acid. The remaining required raw materials, reagents, and preparation methods were the same as in Example 1 to obtain (E) -3- (3- (m-methylphenyl) acryloyl) oxazolidin-2-one (yield 82.1%). ¹H NMR (400 MHz, DMSO-d₆) δ 7.81 (d, J = 16.0 Hz, 1H), 7.72 (d, J = 16.0 Hz, 1H), 7.51-7.44 (m, 2H), 7.36 (dt, J = 10.2, 5.1 Hz, 1H), 7.28 (d, J = 7.8 Hz, 1H), 4.42 (td, J = 8.1, 2.4 Hz, 2H), 4.00 (td, J = 8.1, 2.6 Hz, 2H), 2.34 (s, 3H). LRMS (ESI): 232.09 [M+H]+.

### Example 3 (E) -3- (3- (p-methylphenyl) acryloyl) oxazolidin-2-one

Replace (E) -3- (o-methylphenyl) acrylic acid with (E) -3- (p-methylphenyl) acrylic acid. The remaining required raw materials, reagents, and preparation methods were the same as in Example 1 to obtain (E) -3- (3- (p-methylphenyl) acryloyl) oxazolidin-2-one (yield 85.2%). ¹H NMR (400 MHz, DMSO-d₆) δ 7.77 (d, J = 15.8 Hz, 1H), 7.70 (d, J = 15.9 Hz, 1H), 7.55 (d, J = 8.2 Hz, 2H), 7.26 (d, J = 7.9 Hz, 2H), 4.39 (dd, J = 8.6, 7.4 Hz, 2H), 3.97 (dd, J = 8.6, 7.4 Hz, 2H), 2.32 (s, 3H). LRMS (ESI): 232.09 [M+H]+.

### Example 4 (E) -3- (3- (2-methoxyphenyl) acryloyl) oxazolidin-2-one

Replace (E) -3- (2-methoxyphenyl) acrylic acid with (E) -3- (2-methoxyphenyl) acrylic acid. The remaining required raw materials, reagents, and preparation methods were the same as in Example 1 to obtain (E) -3- (3- (2-methoxyphenyl) acryloyl) oxazolidin-2-one (yield 87.2%). ¹H NMR (400 MHz, DMSO-d6) δ 7.97 (d, J=16.0 Hz, 1H), 7.88 (d, J=15.9 Hz, 1H), 7.61 (dd, J=7.7, 1.6 Hz, 1H), 7.45 (ddd, J=8.5, 7.3, 1.6 Hz, 1H), 7.12 (d, J=8.4 Hz, 1H), 7.03 (t, J=7.5 Hz, 1H), 4.41 (t, J=8.0 Hz, 2H), 3.99 (dd, J=8.5, 7.4 Hz, 2H), 3.88 (s, 3H). LRMS (ESI): 248.08 [M+H]+.

### Example 5 (E) -3- (3- (3-methoxyphenyl) acryloyl) oxazolidin-2-one

Replace (E) -3- (o-methoxyphenyl) acrylic acid with (E) -3- (3-methoxyphenyl) acrylic acid. The remaining required raw materials, reagents, and preparation methods were the same as in Example 1 to obtain (E) -3- (3- (3-methoxyphenyl) acryloyl) oxazolidin-2-one (yield 87.6%). ¹H NMR (400 MHz, DMSO-d₆) δ 7.81 (d, J = 15.8 Hz, 1H), 7.73 (d, J = 15.8 Hz, 1H), 7.39 (t, J = 7.9 Hz, 1H), 7.31 - 7.24 (m, 1H), 7.21 (t, J = 2.0 Hz, 1H), 7.08 - 7.02 (m, 1H), 4.42 (dd, J = 8.5, 7.4 Hz, 2H), 4.00 (dd, J = 8.6, 7.3 Hz, 2H), 3.80 (s, 3H). LRMS (ESI): 248.08 [M+H]+.

### Example 6 (E) -3- (3- (4-methoxyphenyl) acryloyl) oxazolidin-2-one

Replace (E) -3- (o-methoxyphenyl) acrylic acid with (E) -3- (4-methoxyphenyl) acrylic acid. The remaining required raw materials, reagents, and preparation methods were the same as in Example 1 to obtain (E) -3- (3- (4-methoxyphenyl) acryloyl) oxazolidin-2-one (yield 82.7%). ¹H NMR (400 MHz, DMSO-d₆) δ 7.77 - 7.66 (m, 2H), 7.64 (d, J = 8.7 Hz, 2H), 7.03 (d, J = 8.8 Hz, 2H), 4.41 (dd, J = 8.5, 7.4 Hz, 2H), 3.99 (dd, J = 8.5, 7.4 Hz, 2H), 3.81 (s, 3H). LRMS (ESI): 248.08 [M+H]+.

### Example 7 (E) -3- (3- (2- (trifluoromethylphenyl) acryloyl) oxazolidin-2-one

Replace (E) -3- (o-methylphenyl) acrylic acid with (E) -3- (2-trifluoromethylphenyl) acrylic acid. The remaining required raw materials, reagents, and preparation methods were the same as in Example 1 to obtain (E) -3- (3- (2- (trifluoromethylphenyl) acryloyl) oxazolidin-2-one (yield 81.5%). ¹H NMR (400 MHz, DMSO-d₆) δ 7.98 - 7.78 (m, 5H), 7.68 (t, J = 7.6 Hz, 1H), 4.43 (dd, J = 8.5, 7.4 Hz, 2H), 4.02 (dd, J = 8.5, 7.4 Hz, 2H). LRMS (ESI): 286.06 [M+H]+.

### Example 8 (E) -3- (3- (3- (trifluoromethylphenyl) acryloyl) oxazolidin-2-one

Replace (E) -3- (o-methylphenyl) acrylic acid with (E) -3- (3-trifluoromethylphenyl) acrylic acid. The remaining required raw materials, reagents, and preparation methods were the same as in Example 1 to obtain (E) -3- (3- (3- (trifluoromethylphenyl) acryloyl) oxazolidin-2-one (yield 80.3%). ¹H NMR (400 MHz, DMSO-d₆) δ 8.05 - 7.97 (m, 2H), 7.94 - 7.83 (m, 2H), 7.82 (d, J = 7.1 Hz, 1H), 7.71 (t, J = 7.8 Hz, 1H), 4.43 (dd, J = 8.5, 7.4 Hz, 2H), 4.02 (dd, J = 8.5, 7.4 Hz, 2H). LRMS (ESI): 286.06 [M+H]+.

### Example 9 (E) -3- (3- (4- (trifluoromethylphenyl) acryloyl) oxazolidin-2-one

Replace (E) -3- (o-methylphenyl) acrylic acid with (E) -3- (4-trifluoromethylphenyl) acrylic acid. The remaining required raw materials, reagents, and preparation methods were the same as in Example 1 to obtain (E) -3- (3- (4- (trifluoromethylphenyl) acryloyl) oxazolidin-2-one (yield 85.6%). ¹H NMR (400 MHz, DMSO-d₆) δ 7.95 - 7.87 (m, 3H), 7.85 - 7.79 (m, 3H), 4.43 (dd, J = 8.5, 7.4 Hz, 2H), 4.01 (dd, J = 8.6, 7.4 Hz, 2H). LRMS (ESI): 286.06 [M+H]+.

### Example 10 (E) -3- (3- (2-fluorophenyl) acryloyl) oxazolidin-2-one

Replace (E) -3- (ortho methylphenyl) acrylic acid with (E) -3- (2-fluorophenyl) acrylic acid. The remaining required raw materials, reagents, and preparation methods were the same as in Example 1 to obtain (E) -3- (3- (2-fluorophenyl) acryloyl) oxazolidin-2-one (yield 92.1%). ¹H NMR (400 MHz, DMSO-d₆) δ 7.93 (d, J = 16.0 Hz, 1H), 7.77 (d, J = 16.0 Hz, 1H), 7.79 - 7.72 (m, 1H), 7.58 - 7.47 (m, 1H), 7.42 - 7.26 (m, 2H), 4.50 - 4.37 (m, 2H), 4.01 (m, 2H). LRMS (ESI): 236.06 [M+H]+.

### Example 11 (E) -3- (3- (4-fluorophenyl) acryloyl) oxazolidin-2-one

Replace (E) -3- (o-methylphenyl) acrylic acid with (E) -3- (4-fluorophenyl) acrylic acid. The remaining required raw materials, reagents, and preparation methods were the same as in Example 1 to obtain (E) -3- (3- (4-fluorophenyl) acryloyl) oxazolidin-2-one (yield 89.0%). ¹H NMR (400 MHz, DMSO-d₆) δ 7.77 (s, 2H), 7.77 - 7.73 (m, 2H), 7.37 - 7.25 (m, 2H), 4.42 (dd, J = 8.6, 7.3 Hz, 2H), 4.00 (dd, J = 8.5, 7.4 Hz, 2H). LRMS (ESI): 236.06 [M+H]+.

### Example 12 (E) -3- (3- (4-chlorophenyl) acryloyl) oxazolidin-2-one

Replace (E) -3- (4-chlorophenyl) acrylic acid with (E) -3- (4-chlorophenyl) acrylic acid. The remaining required raw materials, reagents, and preparation methods were the same as in Example 1 to obtain (E) -3- (3- (4-chlorophenyl) acryloyl) oxazolidin-2-one (yield 91.1%).¹H NMR (400 MHz, DMSO-d₆) δ 7.82 (d, J = 15.9 Hz, 1H), 7.75 (d, J = 15.9 Hz, 1H), 7.73 - 7.68 (m, 2H), 7.53 (dd, J = 8.4, 1.2 Hz, 2H), 4.42 (t, J = 8.0 Hz, 2H), 4.00 (t, J = 8.0 Hz, 2H). LRMS (ESI): 252.03 [M+H]+.

### Example 13 (E) -3- (3- (2-bromophenyl) acryloyl) oxazolidin-2-one

Replace (E) -3- (ortho methylphenyl) acrylic acid with (E) -3- (2-bromophenyl) acrylic acid. The remaining required raw materials, reagents, and preparation methods were the same as in Example 1 to obtain (E) -3- (3- (2-bromophenyl) acryloyl) oxazolidin-2-one (yield 93.4%). ¹H NMR (400 MHz, DMSO-d₆) δ 7.96 (dd, J = 15.9, 1.7 Hz, 1H), 7.84 - 7.73 (m, 3H), 7.50 (td, J = 7.5, 1.4 Hz, 1H), 7.39 (td, J = 7.7, 1.8 Hz, 1H), 4.43 (td, J = 7.9, 1.7 Hz, 2H), 4.01 (ddd, J = 8.7, 7.2, 1.7 Hz, 2H). LRMS (ESI): 295.98, 297.98 [M+H]+.

### Example 14 (E) -3- (3- (4-bromophenyl) acryloyl) oxazolidin-2-one

Replace (E) -3- (o-methylphenyl) acrylic acid with (E) -3- (4-bromophenyl) acrylic acid. The remaining required raw materials, reagents, and preparation methods were the same as in Example 1 to obtain (E) -3- (3- (4-bromophenyl) acryloyl) oxazolidin-2-one (yield 91.1%).¹H NMR (400 MHz, DMSO-d₆) δ 7.83 (d, J = 15.9 Hz, 1H), 7.73 (d, J = 15.9 Hz, 1H), 7.67 (d, J = 8.7 Hz, 2H), 7.63 (d, J = 8.7 Hz, 2H), 4.42 (dd, J = 8.6, 7.4 Hz, 2H), 4.00 (dd, J = 8.5, 7.4 Hz, 2H). LRMS (ESI): 295.98, 297.98 [M+H]+.

### Example 15 (E) -3- (3- (2-phenoxyphenyl) acryloyl) oxazolidin-2-one

Replace (E) -3- (o-methylphenyl) acrylic acid with (E) -3- (2-phenoxyphenyl) acrylic acid. The remaining required raw materials, reagents, and preparation methods were the same as in Example 1 to obtain (E) -3- (3- (2-phenoxyphenyl) acryloyl) oxazolidin-2-one (yield 89.3%). ¹H NMR (400 MHz, DMSO-d₆) δ 7.99 - 7.88 (m, 2H), 7.83 - 7.76 (m, 1H), 7.50 - 7.44 (m, 1H), 7.41 (tq, J = 7.4, 1.2 Hz, 2H), 7.27 (td, J = 7.6, 1.5 Hz, 1H), 7.17 (tt, J = 7.4, 1.2 Hz, 1H), 7.05 - 6.98 (m, 2H), 6.96 (dt, J = 8.2, 1.3 Hz, 1H), 4.40 (td, J = 8.0, 1.4 Hz, 2H), 4.01 - 3.91 (m, 2H). LRMS (ESI): 310.10 [M+H]+.

### Example 16 (E) -3- (3- (3-phenoxyphenyl) acryloyl) oxazolidin-2-one

Replace (E) -3- (o-methylphenyl) acrylic acid with (E) -3- (3-phenoxyphenyl) acrylic acid. The remaining required raw materials, reagents, and preparation methods were the same as in Example 1 to obtain (E) -3- (3- (3-phenoxyphenyl) acryloyl) oxazolidin-2-one (yield 82.7%). ¹H NMR (400 MHz, DMSO-d₆) δ 7.80 - 7.70 (m, 2H), 7.49 - 7.39 (m, 4H), 7.31 (dd, J = 2.9, 1.4 Hz, 1H), 7.22 - 7.15 (m, 1H), 7.10 - 7.00 (m, 3H), 4.45 - 4.36 (m, 2H), 4.03 - 3.94 (m, 2H). LRMS (ESI): 310.10 [M+H]+.

### Example 17 (E) -3- (3- (4-phenoxyphenyl) acryloyl) oxazolidin-2-one

Replace (E) -3- (o-methylphenyl) acrylic acid with (E) -3- (4-phenoxyphenyl) acrylic acid. The remaining required raw materials, reagents, and preparation methods were the same as in Example 1 to obtain (E) -3- (3- (4-phenoxyphenyl) acryloyl) oxazolidin-2-one (yield 88.1%). ¹H NMR (400 MHz, DMSO-d₆) δ 7.81 (d, J = 15.1 Hz, 1H), 7.61 - 7.52 (m, 1H), 7.54 - 7.45 (m, 2H), 7.39 (t, J = 7.5 Hz, 2H), 7.19 - 7.10 (m, 1H), 7.06 (ddd, J = 7.8, 5.7, 1.9 Hz, 4H), 4.40 (t, J = 6.3 Hz, 2H), 4.05 (t, J = 6.4 Hz, 2H). LRMS (ESI): 310.10 [M+H]+.

### Example 18 (E) -3- (3- ([1,1 '- biphenyl] -2-yl) acryloyl) oxazolidin-2-one

Replace (E) -3- (o-methylphenyl) acrylic acid with (E) -3- ([1,1 '- biphenyl] -2-yl) acrylic acid. The remaining required raw materials, reagents, and preparation methods were the same as in Example 1 to obtain (E) -3- (3- ([1,1' - biphenyl] -2-yl) acryloyl) oxazolidin-2-one (yield 86.7%). ¹H NMR (400 MHz, DMSO-d₆) δ 7.90 - 7.78 (m, 2H), 7.64 (dd, J = 15.8, 1.4 Hz, 1H), 7.60 - 7.39 (m, 6H), 7.33 (dt, J = 7.8, 1.5 Hz, 2H), 4.47 - 4.36 (m, 2H), 3.95 (m, 2H). LRMS (ESI): 294.11 [M+H]+.

### Example 19 (E) -3- (3- ([1,1 '- biphenyl] -3-yl) acryloyl) oxazolidin-2-one

Replace (E) -3- (o-methylphenyl) acrylic acid with (E) -3- ([1,1 '- biphenyl] -3-yl) acrylic acid. The remaining required raw materials, reagents, and preparation methods were the same as in Example 1 to obtain (E) -3- (3- ([1,1' - biphenyl] -3-yl) acryloyl) oxazolidin-2-one (yield 88.3%). ¹H NMR (400 MHz, DMSO-d₆) δ 7.94 (s, 1H), 7.88 (d, J = 5.9 Hz, 2H), 7.78 - 7.68 (m, 4H), 7.57 (t, J = 7.7 Hz, 1H), 7.51 (t, J = 7.6 Hz, 2H), 7.42 (t, J = 7.3 Hz, 1H), 4.43 (t, J = 8.0 Hz, 2H), 4.02 (t, J = 7.9 Hz, 2H). LRMS (ESI): 294.11 [M+H]+.

### Example 20 (E) -3- (3- (4-fluoro-2-trifluoromethylphenyl) acryloyl) oxazolidin-2-one

Replace (E) -3- (o-methylphenyl) acrylic acid with (E) -3- (4-fluoro-2-trifluoromethylphenyl) acrylic acid. The remaining required raw materials, reagents, and preparation methods were the same as in Example 1 to obtain (E) -3- (3- (4-fluoro-2-trifluoromethylphenyl) acryloyl) oxazolidin-2-one (yield 81.0%). ¹H NMR (400 MHz, Chloroform-d) δ 8.15 (dq, J = 15.6, 2.3 Hz, 1H), 7.91 - 7.78 (m, 2H), 7.42 (dd, J = 8.8, 2.7 Hz, 1H), 7.35 - 7.22 (m, 1H), 4.49 (t, J = 8.0 Hz, 2H), 4.16 (t, J = 8.0 Hz, 2H). LRMS (ESI): 304.05 [M+H]+.

### Example 21 (E) -3- (3- (naphthalen-1-yl) acryloyl) oxazolidin-2-one

Replace (E) -3- (ortho methylphenyl) acrylic acid with (E) -3- (naphthalen-1-yl) acrylic acid. The remaining required raw materials, reagents, and preparation methods were the same as in Example 1 to obtain (E) -3- (3- (naphthalen-1-yl) acryloyl) oxazolidin-2-one (yield 87.1%). ¹H NMR (400 MHz, DMSO-d₆) δ 8.52 (d, J = 15.6 Hz, 1H), 8.25 (d, J = 8.3 Hz, 1H), 8.09 - 8.00 (m, 2H), 7.95 - 7.86 (m, 2H), 7.69 - 7.58 (m, 3H), 4.45 (dd, J = 8.6, 7.3 Hz, 2H), 4.05 (dd, J = 8.6, 7.3 Hz, 2H). LRMS (ESI): 268.09 [M+H]+.

### Example 22 (E) -3- (3- (benzo [d] [1,3] dioxolan-5-yl) acryloyl) oxazolidin-2-one

Replace (E) -3- (o-methylphenyl) acrylic acid with (E) -3- (benzo [d] [1,3] dioxolane-5-yl) acrylic acid. The remaining required raw materials, reagents, and preparation methods were the same as in Example 1 to obtain (E) -3- (3- (benzo [d] [1,3] dioxolane-5-yl) acryloyl) oxazolin-2-one (yield 89.2%). ¹H NMR (400 MHz, Chloroform-d) δ 7.79 (d, J = 15.7 Hz, 1H), 7.73 (d, J = 15.6 Hz, 1H), 7.15 (d, J = 1.7 Hz, 1H), 7.10 (dd, J = 8.0, 1.8 Hz, 1H), 6.82 (d, J = 8.0 Hz, 1H), 6.02 (s, 2H), 4.45 (dd, J = 8.6, 7.5 Hz, 2H), 4.13 (dd, J = 8.5, 7.4 Hz, 2H); LRMS (ESI): 262.06 [M + H]⁺.

### Example 23 (E) -3- (3- (thiphen-2-yl) acryloyl) oxazolidin-2-one

Replace (E) -3- (o-methylphenyl) acrylic acid with (E) -3- (thiphen-2-yl) acrylic acid. The remaining required raw materials, reagents, and preparation methods were the same as in Example 1 to obtain (E) -3- (3- (thiphen-2-yl) acryloyl) oxazolidin-2-one (yield 87.4%). ¹H NMR (400 MHz, DMSO-d₆) δ 8.03 (dd, J = 2.8, 1.2 Hz, 1H), 7.77 (d, J = 15.8 Hz, 1H), 7.66 (ddd, J = 5.0, 2.9, 0.7 Hz, 1H), 7.61 (d, J = 15.7 Hz, 1H), 7.42 (dd, J = 5.1, 1.2 Hz, 1H), 4.41 (dd, J = 8.5, 7.4 Hz, 2H), 3.99 (dd, J = 8.5, 7.4 Hz, 2H). LRMS (ESI): 224.03 [M+H]+.

### Example 24 (E) -3- (3-cyclohexylcacryloyl) oxazolidin-2-one

Replace (E) -3- (o-methylphenyl) acrylic acid with (E) -3-cyclohexanoic acid. The remaining required raw materials, reagents, and preparation methods were the same as in Example 1 to obtain (E) -3- (3-cyclohexanoyl) oxazolidin-2-one (yield 85.1%). ¹H NMR (400 MHz, DMSO-d₆) δ 7.12 (dd, J = 15.6, 1.3 Hz, 1H), 6.95 (dd, J = 15.6, 6.6 Hz, 1H), 4.37 (dd, J = 8.5, 7.5 Hz, 2H), 3.92 (dd, J = 8.6, 7.4 Hz, 2H), 2.21 (qd, J = 8.0, 7.3, 4.5 Hz, 1H), 1.77 - 1.68 (m, 4H), 1.63 (d, J = 12.5 Hz, 1H), 1.36 - 1.23 (m, 2H), 1.14 (pd, J = 13.3, 12.7, 3.7 Hz, 3H). LRMS (ESI): 224.12 [M+H]+.

### Example 26 (E) -3- (3- (2-trifluoromethylphenyl) acryloyl) -4-phenyloxazolidin-2-one

Replace (E) -3- (o-methylphenyl) acrylic acid with (E) -3- (2-trifluoromethylphenyl) acrylic acid, and replace oxazolidin-2-one with 4-phenyloxazolidin-2-one. The remaining required raw materials, reagents, and preparation methods were the same as in Example 1 to obtain (E) -3- (3- (2-trifluoromethylphenyl) acryloyl) -4-phenyloxazolidin-2-one (yield 89.9%). ¹H NMR (400 MHz, DMSO-d₆) δ 7.95 (d, J = 7.8 Hz, 1H), 7.91 - 7.77 (m, 4H), 7.68 (t, J = 7.7 Hz, 1H), 7.46 - 7.33 (m, 5H), 5.59 (dd, J = 8.6, 4.1 Hz, 1H), 4.82 (t, J = 8.7 Hz, 1H), 4.24 (dd, J = 8.7, 4.1 Hz, 1H). LRMS (ESI): 362.09 [M+H]+.

### Example 27 (S, E) -3- (3- (2-trifluoromethylphenyl) acryloyl) -4-phenyloxazolidin-2-one

Replace (E) -3- (o-methylphenyl) acrylic acid with (E) -3- (2-trifluoromethylphenyl) acrylic acid, and replace oxazolidin-2-one with (S) -4-phenyloxazolidin-2-one. The remaining required raw materials, reagents, and preparation methods were the same as in Example 1 to obtain (S, E) -3- (3- (2-trifluoromethylphenyl) acryloyl) -4-phenyloxazolidin-2-one (yield 90.9%). ¹H NMR (400 MHz, DMSO-d₆) δ 7.95 (d, J = 7.8 Hz, 1H), 7.91 - 7.77 (m, 4H), 7.68 (t, J = 7.7 Hz, 1H), 7.46 - 7.33 (m, 5H), 5.59 (dd, J = 8.6, 4.1 Hz, 1H), 4.82 (t, J = 8.7 Hz, 1H), 4.24 (dd, J = 8.7, 4.1 Hz, 1H). LRMS (ESI): 362.09 [M+H]+.

### Example 28 (R, E) -3- (3- (2-trifluoromethylphenyl) acryloyl) -4-phenyloxazolidin-2-one

Replace (E) -3- (o-methylphenyl) acrylic acid with (E) -3- (2-trifluoromethylphenyl) acrylic acid, and replace oxazolidin-2-one with (R) -4-phenyloxazolidin-2-one. The remaining required raw materials, reagents, and preparation methods were the same as in Example 1 to obtain (R, E) -3- (3- (2-trifluoromethylphenyl) acryloyl) -4-phenyloxazolidin-2-one (yield 90.4%). ¹H NMR (400 MHz, DMSO-d₆) δ 7.95 (d, J = 7.8 Hz, 1H), 7.91 - 7.77 (m, 4H), 7.68 (t, J = 7.7 Hz, 1H), 7.46 - 7.33 (m, 5H), 5.59 (dd, J = 8.6, 4.1 Hz, 1H), 4.82 (t, J = 8.7 Hz, 1H), 4.24 (dd, J = 8.7, 4.1 Hz, 1H). LRMS (ESI): 362.09 [M+H]+.

### Example 29 (S, E) -4-phenyl-3- (3- (3- (trifluoromethoxy) phenyl) acryloyl) oxazolidin-2-one

Replace (E) -3- (o-methylphenyl) acrylic acid with (E) - (3- (trifluoromethoxy) phenyl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (trifluoromethoxy) phenyl) acryloyl) oxazolidin-2-one. ¹H NMR (400 MHz, Chloroform-d) δ 7.94 (d, J = 15.7 Hz, 1H), 7.72 (d, J = 15.7 Hz, 1H), 7.52 (dt, J = 7.8, 1.3 Hz, 1H), 7.45 - 7.32 (m, 7H), 7.25 (ddd, J = 8.2, 2.7, 1.5 Hz, 1H), 5.56 (dd, J = 8.7, 3.9 Hz, 1H), 4.75 (t, J = 8.8 Hz, 1H), 4.33 (dd, J = 8.9, 3.9 Hz, 1H) ; LRMS (ESI): 378.09 [M + H]⁺.

### Example 30 (S, E) -4-phenyl-3- (3- (2- (trifluoromethoxy) phenyl) acryloyl) oxazolidin-2-one

Replace (E) -3- (o-methylphenyl) acrylic acid with (E) - (2- (trifluoromethoxy) phenyl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (2- (trifluoromethoxy) phenyl) acryloyl) oxazolidin-2-one. ¹H NMR (400 MHz, Chloroform-d) δ 8.05 (d, J = 15.8 Hz, 1H), 7.97 (d, J = 15.9 Hz, 1H), 7.84 - 7.75 (m, 1H), 7.47 - 7.25 (m, 8H), 5.56 (dd, J = 8.8, 3.9 Hz, 1H), 4.76 (t, J = 8.8 Hz, 1H), 4.34 (dd, J = 8.9, 3.9 Hz, 1H); LRMS (ESI): 378.09 [M + H]⁺.

### Example 31 (S, E) -4-phenyl-3- (3- (4- (trifluoromethoxy) phenyl) acryloyl) oxazolidin-2-one

Replace (E) -3- (o-methylphenyl) acrylic acid with (E) - (4- (trifluoromethoxy) phenyl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (4- (trifluoromethoxy) phenyl) acryloyl) oxazolidin-2-one. ¹H NMR (400 MHz, Methylene Chloride-d₂) δ 7.90 (d, J = 15.8 Hz, 1H), 7.73 (d, J = 15.7 Hz, 1H), 7.68 - 7.63 (m, 2H), 7.44 - 7.32 (m, 5H), 7.28 - 7.22 (m, 2H), 5.53 (dd, J = 8.7, 4.1 Hz, 1H), 4.74 (t, J = 8.8 Hz, 1H), 4.29 (dd, J = 8.9, 4.1 Hz, 1H); LRMS (ESI): 378.09 [M + H]⁺.

### Example 32 (S, E) -3- (3- (2-phenoxyphenyl) acryloyl) -4-phenyloxazolidin-2-one

Replace (E) -3- (o-methylphenyl) acrylic acid with (E) -3- (2-phenoxyphenyl) acrylic acid, and replace oxazolidin-2-one with (S) -4-phenyloxazolidin-2-one. The remaining required raw materials, reagents, and preparation methods were the same as in Example 1 to obtain (S, E) -3- (3- (2-phenoxyphenyl) acryloyl) -4-phenyloxazolidin-2-one (yield 90.1%). ¹H NMR (400 MHz, DMSO-d₆) δ 7.98 (d, J = 15.9 Hz, 1H), 7.84 (d, J = 16.0 Hz, 1H), 7.80 (dd, J = 7.9, 1.7 Hz, 1H), 7.47 (ddd, J = 8.3, 7.3, 1.7 Hz, 1H), 7.43 - 7.36 (m, 4H), 7.35 - 7.25 (m, 4H), 7.18 - 7.13 (m, 1H), 7.03 - 6.97 (m, 2H), 6.94 (dd, J = 8.2, 1.1 Hz, 1H), 5.55 (dd, J = 8.6, 3.9 Hz, 1H), 4.79 (t, J = 8.7 Hz, 1H), 4.20 (dd, J = 8.7, 3.9 Hz, 1H). LRMS (ESI): 386.13 [M+H]+.

### Example 33 (R, E) -3- (3- (2-phenoxyphenyl) acryloyl) -4-phenyloxazolidin-2-one

Replace (E) -3- (o-methylphenyl) acrylic acid with (E) -3- (2-phenoxyphenyl) acrylic acid, and replace oxazolidin-2-one with (R) -4-phenyloxazolidin-2-one. The remaining required raw materials, reagents, and preparation methods were the same as in Example 1 to obtain (R, E) -3- (3- (2-phenoxyphenyl) acryloyl) -4-phenyloxazolidin-2-one (yield 90.5%). ¹H NMR (400 MHz, DMSO-d₆) δ 7.98 (d, J = 15.9 Hz, 1H), 7.84 (d, J = 16.0 Hz, 1H), 7.80 (dd, J = 7.9, 1.7 Hz, 1H), 7.47 (ddd, J = 8.3, 7.3, 1.7 Hz, 1H), 7.43 - 7.36 (m, 4H), 7.35 - 7.25 (m, 4H), 7.18 - 7.13 (m, 1H), 7.03 - 6.97 (m, 2H), 6.94 (dd, J = 8.2, 1.1 Hz, 1H), 5.55 (dd, J = 8.6, 3.9 Hz, 1H), 4.79 (t, J = 8.7 Hz, 1H), 4.20 (dd, J = 8.7, 3.9 Hz, 1H). LRMS (ESI): 386.13 [M+H]+.

### Example 34 (E) -3- (3- ([1,1 '- biphenyl] -3-yl) acryloyl) -4-phenyloxazolidin-2-one

Replace (E) -3- (o-methylphenyl) acrylic acid with (E) -3- ([1,1 '- biphenyl] -3-yl) acrylic acid, replace oxazolidin-2-one with 4-phenyloxazolidin-2-one. The remaining required raw materials, reagents, and preparation methods were the same as in Example 1 to obtain (E) -3- (3- ([1,1' - biphenyl] -3-yl) acryloyl) -4-phenyloxazolidin-2-one (yield 86.9%). ¹H NMR (400 MHz, DMSO-d₆) δ 7.96 - 7.89 (m, 2H), 7.81 - 7.67 (m, 5H), 7.56 (t, J = 7.7 Hz, 1H), 7.50 (dd, J = 8.2, 6.9 Hz, 2H), 7.44 - 7.32 (m, 6H), 5.61 (dd, J = 8.6, 3.9 Hz, 1H), 4.82 (t, J = 8.7 Hz, 1H), 4.23 (dd, J = 8.6, 3.9 Hz, 1H). LRMS (ESI, m/z): 370.14 [M+H]⁺

### Example 35 (S, E) -3- (3- ([1,1 '- biphenyl] -3-yl) acryloyl) -4-phenyloxazolidin-2-one

Replace (E) -3- (o-methylphenyl) acrylic acid with (E) -3- ([1,1 '- biphenyl] -3-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- ([1,1' - biphenyl] -3-yl) acryloyl) -4-phenyloxazolidin-2-one (yield 89.6%). ¹H NMR (400 MHz, DMSO-d₆) δ 8.00 - 7.88 (m, 2H), 7.81 - 7.66 (m, 5H), 7.56 (t, J = 7.7 Hz, 1H), 7.53 - 7.47 (m, 2H), 7.45 - 7.33 (m, 6H), 5.61 (dd, J = 8.6, 3.9 Hz, 1H), 4.87 - 4.78 (m, 1H), 4.27 - 4.20 (m, 1H). LRMS (ESI): 370.14 [M+H]+.

### Example 36 (R, E) -3- (3- ([1,1 '- biphenyl] -3-yl) acryloyl) -4-phenyloxazolidin-2-one

Replace (E) -3- (o-methylphenyl) acrylic acid with (E) -3- ([1,1 '- biphenyl] -3-yl) acrylic acid, and replace oxazolidin-2-one with (R) -4-phenyloxazolidin-2-one. The remaining required raw materials, reagents, and preparation methods were the same as in Example 1 to obtain (R, E) -3- (3- ([1,1' - biphenyl] -3-yl) acryloyl) -4-phenyloxazolidin-2-one (yield 88.9%). ¹H NMR (400 MHz, DMSO-d₆) δ 8.00 - 7.88 (m, 2H), 7.81 - 7.66 (m, 5H), 7.56 (t, J = 7.7 Hz, 1H), 7.53 - 7.47 (m, 2H), 7.45 - 7.33 (m, 6H), 5.61 (dd, J = 8.6, 3.9 Hz, 1H), 4.87 - 4.78 (m, 1H), 4.27 - 4.20 (m, 1H). LRMS (ESI): 370.14 [M+H]+.

### Example 37 (S, E) -3- (3- (4 '- fluorine - [1,1' - biphenyl] -3-yl) acryloyl) -4-phenyloxazolidin-2-one

Replace (E) -3- (o-methylphenyl) acrylic acid with (E) -3- (4 '- fluorine - [1,1' - biphenyl] -3-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (4 '- fluorine - [1,1' - biphenyl] -3-yl) acryloyl) -4-phenyloxazolidin-2-one (yield 78.1%).¹H NMR (400 MHz, DMSO-d₆) δ 7.94 - 7.87 (m, 2H), 7.80 - 7.70 (m, 4H), 7.68 (dt, J = 7.9, 1.3 Hz, 1H), 7.55 (t, J = 7.7 Hz, 1H), 7.43 - 7.39 (m, 1H), 7.39 - 7.36 (m, 2H), 7.36 - 7.28 (m, 4H), 5.61 (dd, J = 8.6, 3.9 Hz, 1H), 4.82 (t, J = 8.7 Hz, 1H), 4.23 (dd, J = 8.6, 3.9 Hz, 1H). LRMS (ESI): 409.9 [M+Na]⁺.

### Example 38 (S, E) -4-phenyl-3- (3- (3- (pyridin-3-yl) phenyl) acryloyl) oxazolidin-2-one

Replace (E) -3- (o-methylphenyl) acrylic acid with (E) -3- (3- (pyridin-3-yl) phenyl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (pyridin-3-yl) phenyl) acryloyl) oxazolidin-2-one (yield 67.1%).¹H NMR (400 MHz, DMSO-d₆) δ 8.95 (d, J = 2.4 Hz, 1H), 8.61 (dd, J = 4.8, 1.6 Hz, 1H), 8.13 (dt, J = 8.0, 2.0 Hz, 1H), 8.01 (t, J = 1.7 Hz, 1H), 7.93 (d, J = 15.9 Hz, 1H), 7.85 - 7.72 (m, 3H), 7.61 (t, J = 7.8 Hz, 1H), 7.52 (dd, J = 8.0, 4.8 Hz, 1H), 7.45 - 7.31 (m, 5H), 5.61 (dd, J = 8.6, 3.9 Hz, 1H), 4.82 (t, J = 8.6 Hz, 1H), 4.23 (dd, J = 8.6, 3.9 Hz, 1H). LRMS (ESI): 393.01 [M+Na]⁺.

### Example 39 (S, E) -4-phenyl-3- (3- (3- (pyridin-4-yl) phenyl) acryloyl) oxazolidin-2-one

Replace (E) -3- (o-methylphenyl) acrylic acid with (E) -3- (3- (pyridin-4-yl) phenyl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (pyridin-4-yl) phenyl) acryloyl) oxazolidin-2-one (yield 65.5%).¹H NMR (400 MHz, DMSO-d₆) δ 8.70 - 8.65 (m, 2H), 8.07 (s, 1H), 7.97 - 7.90 (m, 1H), 7.89 (d, J = 7.4 Hz, 1H), 7.82 - 7.73 (m, 4H), 7.62 (t, J = 7.6 Hz, 1H), 7.45 - 7.31 (m, 5H), 5.61 (dd, J = 8.6, 3.9 Hz, 1H), 4.82 (t, J = 8.6 Hz, 1H), 4.23 (dd, J = 8.6, 3.9 Hz, 1H). LRMS (ESI): 392.92 [M+Na]⁺.

### Example 40 (S, E) -4-phenyl-3- (3- (3- (thiphen-2-yl) phenyl) acryloyl) oxazolidin-2-one

Replace (E) -3- (o-methylphenyl) acrylic acid with (E) -3- (3- (thiphen-2-yl) phenyl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (thiphen-2-yl) phenyl) acryloyl) oxazolidin-2-one (yield 87.8%). ¹H NMR (400 MHz, DMSO-d₆) δ 7.81 (d, J = 15.0 Hz, 1H), 7.78 - 7.66 (m, 2H), 7.62 (q, J = 1.7 Hz, 1H), 7.55 (d, J = 15.2 Hz, 1H), 7.55 - 7.43 (m, 2H), 7.45 - 7.35 (m, 3H), 7.36 - 7.26 (m, 2H), 7.29 - 7.19 (m, 1H), 7.13 (t, J = 7.5 Hz, 1H), 5.29 (t, J = 7.0 Hz, 1H), 5.08 (dd, J = 11.5, 7.0 Hz, 1H), 4.80 (dd, J = 11.5, 7.0 Hz, 1H). LRMS (ESI): 376.09 [M+H]+.

### Example 41 (S, E) -4-phenyl-3- (3- (5-phenylthiophen-2-yl) acryloyl) oxazolidin-2-one

Replace (E) -3- (o-methylphenyl) acrylic acid with (E) -3- (5-phenylthiphen-2-yl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (5-phenylthiphen-2-yl) acryloyl) oxazolidin-2-one (yield 91.3%). ¹H NMR (400 MHz, DMSO-d₆) δ 7.89 (d, J = 7.6 Hz, 1H), 7.87 - 7.77 (m, 3H), 7.71 (d, J = 7.4 Hz, 1H), 7.55 (d, J = 15.1 Hz, 1H), 7.52 - 7.44 (m, 3H), 7.47 - 7.38 (m, 2H), 7.31 (t, J = 7.3 Hz, 2H), 7.29 - 7.19 (m, 1H), 5.29 (t, J = 7.0 Hz, 1H), 5.07 (dd, J = 11.5, 7.0 Hz, 1H), 4.81 (dd, J = 11.4, 6.9 Hz, 1H). LRMS (ESI): 376.09 [M+H]+.

### Example 42 (S, E) -4-phenyl-3- (3- (4-phenylthiophen-2-yl) acryloyl) oxazolidin-2-one

Replace (E) -3- (o-methylphenyl) acrylic acid with (E) -3- (4-phenylthiphen-2-yl) acrylic acid, and replace oxazolidin-2-one with (S) -4-phenyloxazolidin-2-one. The remaining required raw materials, reagents, and preparation methods were the same as in Example 1 to obtain (S, E) -4-phenyl-3- (3- (4-phenylthiphen-2-yl) acryloyl) oxazolidin-2-one (yield 92.1%). ¹H NMR (400 MHz, DMSO-d₆) δ 7.86 - 7.77 (m, 3H), 7.63 - 7.53 (m, 3H), 7.42 (dtdd, J = 9.3, 7.1, 4.5, 3.0 Hz, 5H), 7.31 (t, J = 7.3 Hz, 2H), 7.29 - 7.19 (m, 1H), 5.31 (t, J = 6.9 Hz, 1H), 5.08 (dd, J = 11.4, 6.9 Hz, 1H), 4.81 (dd, J = 11.5, 7.0 Hz, 1H). LRMS (ESI): 376.09 [M+H]+.

### Example 43 (S, E) -3- (3- (4-fluoro-2- (trifluoromethyl) phenyl) acryloyl) -4-phenyloxazolidin-2-one

Replace (E) -3- (o-methylphenyl) acrylic acid with (E) -4-fluoro-2- (trifluoromethyl) phenyl) acrylic acid, and replace oxazolidin-2-one with (S) -4-phenyloxazolidin-2-one. The remaining required raw materials, reagents, and preparation methods were the same as in Example 1 to obtain (S, E) -3- (3- (4-fluoro-2- (trifluoromethyl) phenyl) acryloyl) -4-phenyloxazolidin-2-one. ¹H NMR (400 MHz, Chloroform-d) δ 8.08 (d, J = 15.4 Hz, 1H), 7.95 - 7.80 (m, 2H), 7.39 (dt, J = 15.8, 7.4 Hz, 6H), 7.32 - 7.22 (m, 1H), 5.55 (dd, J = 8.7, 3.9 Hz, 1H), 4.76 (t, J = 8.8 Hz, 1H), 4.35 (dd, J = 8.9, 3.9 Hz, 1H); LRMS (ESI): 380.24 [M + H]⁺.

### Example 44 (S, E) -3- (3- (benzo [d] [1,3] dioxolan-5-yl) acryloyl) -4-phenyloxazolidin-2-one

Replace (E) -3- (o-methylphenyl) acrylic acid with (E) -3- (benzo [d] [1,3] dioxolan-5-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (benzo [d] [1,3] dioxolan-5-yl) acryloyl) -4-phenyloxazolidin-2-one. ¹H NMR (400 MHz, Chloroform-d) δ 7.84 - 7.64 (m, 2H), 7.44 - 7.29 (m, 5H), 7.13 (d, J = 1.7 Hz, 1H), 7.05 (dd, J = 8.1, 1.7 Hz, 1H), 6.80 (d, J = 8.0 Hz, 1H), 6.01 (s, 2H), 5.55 (dd, J = 8.7, 3.9 Hz, 1H), 4.73 (t, J = 8.8 Hz, 1H), 4.31 (dd, J = 8.8, 3.8 Hz, 1H); LRMS (ESI): 338.10 [M + H]⁺.

### Example 45 (S, E) -3- (3- (2-chloroquinolin-4-yl) acryloyl) -4-phenyloxazolidin-2-one

Replace (E) -3- (o-methylphenyl) acrylic acid with (E) -3- (2-chloroquinolin-4-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (2-chloroquinolin-4-yl) acryloyl) -4-phenyloxazolidin-2-one. ¹H NMR (400 MHz, Chloroform-d) δ 8.38 (dd, J = 15.6, 0.8 Hz, 1H), 8.13 - 8.05 (m, 3H), 7.77 (ddd, J = 8.4, 6.9, 1.4 Hz, 1H), 7.64 (s, 1H), 7.63 - 7.58 (m, 1H), 7.40 (m, 5H), 5.59 (dd, J = 8.8, 4.0 Hz, 1H), 4.81 (t, J = 8.8 Hz, 1H), 4.43 - 4.35 (m, 1H); LRMS (ESI): 379.08 [M + H]⁺.

### Example 46 (S, E) -3- (3- (benzofuran-2-yl) acryloyl) -4-phenyloxazolidin-2-one

Replace (E) -3- (o-methylphenyl) acrylic acid with (E) -3- (benzofuran-2-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (benzofuran-2-yl) acryloyl) -4-phenyloxazolidin-2-one. ¹H NMR (400 MHz, Methylene Chloride-d₂) δ 7.95 (d, J = 15.4 Hz, 1H), 7.67 - 7.58 (m, 2H), 7.55 (dt, J = 8.4, 1.0 Hz, 1H), 7.45 - 7.32 (m, 6H), 7.30 - 7.22 (m, 1H), 7.03 (d, J = 4.6 Hz, 1H), 5.54 (dd, J = 8.8, 4.1 Hz, 1H), 4.75 (t, J = 8.8 Hz, 1H), 4.29 (dd, J = 8.9, 4.1 Hz, 1H); LRMS (ESI): 334.10 [M + H]⁺.

### Example 47 (S, E) -3- (3- (benzofuran-7-yl) acryloyl) -4-phenyloxazolidin-2-one

Replace (E) -3- (o-methylphenyl) acrylic acid with (E) -3- (benzofuran-7-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (benzofuran-7-yl) acryloyl) -4-phenyloxazolidin-2-one. ¹H NMR (400 MHz, Chloroform-d) δ 7.97 (d, J = 15.7 Hz, 1H), 7.92 (d, J = 15.7 Hz, 1H), 7.86 (d, J = 1.7 Hz, 1H), 7.67 (d, J = 2.2 Hz, 1H), 7.60 (dd, J = 8.6, 1.8 Hz, 1H), 7.52 (d, J = 8.6 Hz, 1H), 7.46 - 7.35 (m, 5H), 6.82 (dd, J = 2.3, 0.9 Hz, 1H), 5.60 (dd, J = 8.7, 3.9 Hz, 1H), 4.78 (t, J = 8.8 Hz, 1H), 4.35 (dd, J = 8.8, 3.9 Hz, 1H) ; LRMS (ESI): 334.10 [M + H]⁺.

### Example 48 (S, E) -3- (3- (naphthalen-1-yl) acryloyl) -4-phenyloxazolidin-2-one

Replace (E) -3- (ortho methylphenyl) acrylic acid with (E) -3- (naphthalen-1-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (naphthalen-1-yl) acryloyl) -4-phenyloxazolidin-2-one. ¹H NMR (400 MHz, Chloroform-d) δ 8.68 (d, J = 15.5 Hz, 1H), 8.20 (d, J = 8.1 Hz, 1H), 8.05 (dt, J = 15.5, 0.9 Hz, 1H), 7.97 - 7.91 (m, 2H), 7.91 - 7.86 (m, 1H), 7.60 - 7.50 (m, 3H), 7.48 - 7.35 (m, 5H), 5.63 (dd, J = 8.7, 3.9 Hz, 1H), 4.80 (ddt, J = 9.7, 8.8, 1.0 Hz, 1H), 4.41 - 4.34 (m, 1H); LRMS (ESI): 344.12 [M + H]⁺.

### Example 49 (S, E) -3- (3- (benzo [b] thiophen-2-yl) acryloyl) -4-phenyloxazolidin-2-one

Replace (E) -3- (o-methylphenyl) acrylic acid with (E) -3- (benzo [b] thiophen-2-yl) acrylic acid, and replace oxazolidin-2-one with (S) -4-phenyloxazolidin-2-one. The remaining required raw materials, reagents, and preparation methods were the same as in Example 1 to obtain (S, E) -3- (3- (benzo [b] thiophen-2-yl) acryloyl) -4-phenyloxazolidin-2-one. ¹H NMR (400 MHz, DMSO-d₆) δ 8.05 - 7.95 (m, 2H), 7.93 - 7.82 (m, 2H), 7.69 (d, J = 15.5 Hz, 1H), 7.39 (dp, J = 21.3, 8.2, 7.0 Hz, 7H), 5.59 (dd, J = 8.7, 3.9 Hz, 1H), 4.81 (t, J = 8.7 Hz, 1H), 4.22 (dd, J = 8.7, 3.9 Hz, 1H); LRMS (ESI): 350.08 [M + H]⁺.

### Example 50 (E) -4- (4 '- fluorophenyl) -3- (3- (2- (trifluoromethylphenyl) acryloyl) oxazolidin-2-one

Replace (E) -3- (o-methylphenyl) acrylic acid with (E) -3- (2-trifluoromethylphenyl) acrylic acid, and replace oxazolin-2-one with 4- (4 '- fluorophenyl) oxazolin-2-one. The remaining required raw materials, reagents, and preparation methods were the same as in Example 1 to obtain (E) -4- (4' - fluorophenyl) -3- (3- (2- (trifluoromethylphenyl) acryloyl) oxazolin-2-one. LRMS (ESI): 380.08 [M + H]+.

### Example 51 (S, E) -4- (4 '- fluorophenyl) -3- (3- (2- (trifluoromethylphenyl) acryloyl) oxazolidin-2-one

Replace (E) -3- (o-methylphenyl) acrylic acid with (E) -3- (2-trifluoromethylphenyl) acrylic acid, and replace oxazolidin-2-one with (S) -4- (4 '- fluorophenyl) oxazolidin-2-one. The remaining required raw materials, reagents, and preparation methods were the same as in Example 1 to obtain (S, E) -4- (4' - fluorophenyl) -3- (3- (2- (trifluoromethylphenyl) acryloyl) oxazolidin-2-one. LRMS (ESI): 380.08 [M + H]⁺.

### Example 52 (E) -4- (4 '- fluorophenyl) -3- (3- (2- (trifluoromethoxyphenyl) acryloyl) oxazolidin-2-one

Replace (E) -3- (o-methylphenyl) acrylic acid with (E) -3- (2-trifluoromethoxyphenyl) acrylic acid, and replace oxazolidin-2-one with 4- (4 '- fluorophenyl) oxazolidin-2-one. The remaining required raw materials, reagents, and preparation methods were the same as in Example 1 to obtain (E) -4- (4' - fluorophenyl) -3- (3- (2- (trifluoromethoxyphenyl) acryloyl) oxazolidin-2-one. LRMS (ESI): 396.08 [M + H]⁺.

### Example 53 (S, E) -4- (4 '- fluorophenyl) -3- (3- (2- (trifluoromethoxyphenyl) acryloyl) oxazolidin-2-one

Replace (E) -3- (o-methylphenyl) acrylic acid with (E) -3- (2-trifluoromethoxyphenyl) acrylic acid, and replace oxazolidin-2-one with (S) -4- (4 '- fluorophenyl) oxazolidin-2-one. The remaining required raw materials, reagents, and preparation methods were the same as in Example 1 to obtain (S, E) -4- (4' - fluorophenyl) -3- (3- (2- (trifluoromethoxyphenyl) acryloyl) oxazolidin-2-one. LRMS (ESI): 396.08 [M + H]⁺.

### Example 54 (E) -4- (4 '- fluorophenyl) -3- (3- (2-phenoxyphenyl) acryloyl) oxazolidin-2-one

Replace (E) -3- (o-methylphenyl) acrylic acid with (E) -3- (2-phenoxyphenyl) acrylic acid, and replace oxazolin-2-one with 4- (4 '- fluorophenyl) oxazolin-2-one. The remaining required raw materials, reagents, and preparation methods were the same as in Example 1 to obtain (E) -4- (4' - fluorophenyl) -3- (3- (2-phenoxyphenyl) acryloyl) oxazolin-2-one. LRMS (ESI): 404.12 [M + H]⁺.

### Example 55 (S, E) -4- (4 '- fluorophenyl) -3- (3- (2-phenoxyphenyl) acryloyl) oxazolidin-2-one

Replace (E) -3- (o-methylphenyl) acrylic acid with (E) -3- (2-phenoxyphenyl) acrylic acid, and replace oxazolidin-2-one with (S) -4- (4 '- fluorophenyl) oxazolidin-2-one. The remaining required raw materials, reagents, and preparation methods were the same as in Example 1 to obtain (S, E) -4- (4' - fluorophenyl) -3- (3- (2-phenoxyphenyl) acryloyl) oxazolidin-2-one. LRMS (ESI): 404.12 [M + H]⁺.

### Example 56 (E) -3- (3- ([1,1 '- biphenyl] -3-yl) acryloyl) -4- (4' - fluorophenyl) oxazolidin-2-one

Replace (E) -3- (o-methylphenyl) acrylic acid with (E) -3- ([1,1 '- biphenyl] -3-yl) acrylic acid, and replace oxazolidin-2-one with 4- (4' - fluorophenyl) oxazolidin-2-one. The remaining required raw materials, reagents, and preparation methods were the same as in Example 1 to obtain (E) -3- (3- ([1,1 '- biphenyl] -3-yl) acryloyl) -4- (4' - fluorophenyl) oxazolidin-2-one. LRMS (ESI): 388.13 [M + H]⁺.

### Example 57 (E) -4-benzyl-3- (3- (2-trifluoromethylphenyl) acryloyl) oxazolidin-2-one

Replace (E) -3- (o-methylphenyl) acrylic acid with (E) -3- (2-trifluoromethylphenyl) acrylic acid, and replace oxazolin-2-one with 4-benzyloxazolin-2-one. The remaining required raw materials, reagents, and preparation methods were the same as in Example 1 to obtain (E) -4-benzyl-3- (3- (2-trifluoromethylphenyl) acryloyl) oxazolin-2-one (yield 86.9%).¹H NMR (400 MHz, DMSO-d₆) δ 7.91 - 7.82 (m, 2H), 7.76 (td, J = 7.4, 2.0 Hz, 1H), 7.64 (d, J = 15.0 Hz, 1H), 7.49 (td, J = 7.5, 2.1 Hz, 1H), 7.33 - 7.23 (m, 3H), 7.24 - 7.14 (m, 3H), 4.64 (p, J = 7.0 Hz, 1H), 4.56 (dd, J = 11.3, 7.0 Hz, 1H), 4.33 (dd, J = 11.3, 6.8 Hz, 1H), 3.17 (dd, J = 12.4, 6.9 Hz, 1H), 2.91 (dd, J = 12.4, 6.9 Hz, 1H). LRMS (ESI): 376.11 [M+H]⁺.

### Example 58 (S, E) -4-benzyl-3- (3- (2-trifluoromethylphenyl) acryloyl) oxazolidin-2-one

Replace (E) -3- (o-methylphenyl) acrylic acid with (E) -3- (2-trifluoromethylphenyl) acrylic acid, and replace oxazolin-2-one with (S) -4-benzyloxazolin-2-one. The remaining required raw materials, reagents, and preparation methods were the same as in Example 1 to obtain (S, E) -4-benzyl-3- (3- (2-trifluoromethylphenyl) acryloyl) oxazolin-2-one (yield 86.9%). ¹H NMR (400 MHz, DMSO-d₆) δ 7.91 - 7.82 (m, 2H), 7.76 (td, J = 7.4, 2.0 Hz, 1H), 7.64 (d, J = 15.0 Hz, 1H), 7.49 (td, J = 7.5, 2.1 Hz, 1H), 7.33 - 7.23 (m, 3H), 7.24 - 7.14 (m, 3H), 4.64 (p, J = 7.0 Hz, 1H), 4.56 (dd, J = 11.3, 7.0 Hz, 1H), 4.33 (dd, J = 11.3, 6.8 Hz, 1H), 3.17 (dd, J = 12.4, 6.9 Hz, 1H), 2.91 (dd, J = 12.4, 6.9 Hz, 1H). LRMS (ESI): 376.11 [M+H]⁺.

### Example 59 (R, E) -4-benzyl-3- (3- (2-trifluoromethylphenyl) acryloyl) oxazolidin-2-one

Replace (E) -3- (o-methylphenyl) acrylic acid with (E) -3- (2-trifluoromethylphenyl) acrylic acid, and replace oxazolin-2-one with (R) -4-benzyloxazolin-2-one. The remaining required raw materials, reagents, and preparation methods were the same as in Example 1 to obtain (R, E) -4-benzyl-3- (3- (2-trifluoromethylphenyl) acryloyl) oxazolin-2-one (yield 86.4%).¹H NMR (400 MHz, DMSO-d₆) δ 7.91 - 7.82 (m, 2H), 7.76 (td, J = 7.4, 2.0 Hz, 1H), 7.64 (d, J = 15.0 Hz, 1H), 7.49 (td, J = 7.5, 2.1 Hz, 1H), 7.33 - 7.23 (m, 3H), 7.24 - 7.14 (m, 3H), 4.64 (p, J = 7.0 Hz, 1H), 4.56 (dd, J = 11.3, 7.0 Hz, 1H), 4.33 (dd, J = 11.3, 6.8 Hz, 1H), 3.17 (dd, J = 12.4, 6.9 Hz, 1H), 2.91 (dd, J = 12.4, 6.9 Hz, 1H). LRMS (ESI): 376.11 [M+H]⁺.

### Example 60 (E) -3- (3- ([1,1 '- biphenyl] -3-yl) acryloyl) -4-benzyloxazolidin-2-one

Replace (E) -3- (o-methylphenyl) acrylic acid with (E) -3- ([1,1 '- biphenyl] -3-yl) acrylic acid, and replace oxazolidin-2-one with 4-benzyloxazolidin-2-one. The remaining required raw materials, reagents, and preparation methods were the same as in Example 1 to obtain (E) -3- (3- ([1,1' - biphenyl] -3-yl) acryloyl) -4-benzyloxazolidin-2-one (yield 79.0%). ¹H NMR (400 MHz, DMSO-d₆) δ 7.95 (s, 1H), 7.93 (d, J = 15.8 Hz, 1H), 7.87 (d, J = 15.8 Hz, 1H), 7.78 - 7.74 (m, 1H), 7.74 - 7.68 (m, 3H), 7.57 (t, J = 7.7 Hz, 1H), 7.50 (dd, J = 8.2, 7.0 Hz, 2H), 7.44 - 7.38 (m, 1H), 7.36 - 7.30 (m, 2H), 7.29 - 7.21 (m, 3H), 4.79 (td, J = 7.7, 3.8 Hz, 1H), 4.40 (t, J = 8.5 Hz, 1H), 4.24 (dd, J = 8.7, 2.9 Hz, 1H), 3.11 (dd, J = 13.6, 3.4 Hz, 1H), 3.02 (dd, J = 13.6, 7.5 Hz, 1H). LRMS (ESI): 384.0 [M+H]⁺.

### Example 61 (S, E) -4-isopropyl-3- (3- (2- (trifluoromethyl) phenyl) acryloyl) oxazolidin-2-one

Replace (E) -3- (o-methylphenyl) acrylic acid with (E) -3- (2-trifluoromethylphenyl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (2- (trifluoromethylphenyl) acryloyl) oxazolidin-2-one. ¹H NMR (400 MHz, Chloroform-d) δ 8.22 (dq, J = 15.5, 2.3 Hz, 1H), 7.92 (d, J = 15.5 Hz, 1H), 7.86 (d, J = 7.8 Hz, 1H), 7.71 (dd, J = 7.8, 1.3 Hz, 1H), 7.58 (td, J = 7.8, 1.5 Hz, 1H), 7.49 (tt, J = 7.6, 1.0 Hz, 1H), 4.60 - 4.54 (m, 1H), 4.34 (dd, J = 9.2, 8.3 Hz, 1H), 4.27 (dd, J = 9.1, 3.2 Hz, 1H), 2.49 (pd, J = 7.0, 3.9 Hz, 1H), 0.95 (dd, J = 15.0, 7.0 Hz, 6H); LRMS (ESI): 328.11 [M + H]⁺.

### Example 62 (S, E) -4-isopropyl-3- (3- (4- (trifluoromethyl) phenyl) acryloyl) oxazolidin-2-one

Replace (E) -3- (o-methylphenyl) acrylic acid with (E) -3- (4-trifluoromethylphenyl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (4- (trifluoromethylphenyl) acryloyl) oxazolidin-2-one. LRMS (ESI): 328.11 [M + H]⁺.

### Example 63 (S, E) -4-isopropyl-3- (3- (3- (trifluoromethyl) phenyl) acryloyl) oxazolidin-2-one

Replace (E) -3- (o-methylphenyl) acrylic acid with (E) -3- (3-trifluoromethylphenyl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (trifluoromethylphenyl) acryloyl) oxazolidin-2-one. LRMS (ESI): 328.11 [M + H]⁺.

### Example 64 (S, E) -4-isopropyl-3- (3- (3-trifluoromethoxyphenyl) acryloyl) oxazolidin-2-one

Replace (E) -3- (o-methylphenyl) acrylic acid with (E) -3- (3-trifluoromethoxyphenyl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (3-trifluoromethoxyphenyl) acryloyl) oxazolidin-2-one. ¹H NMR (400 MHz, Chloroform-d) δ 7.96 (d, J = 15.7 Hz, 1H), 7.80 (d, J = 15.7 Hz, 1H), 7.56 (dt, J = 7.8, 1.2 Hz, 1H), 7.47 - 7.40 (m, 2H), 7.29 - 7.21 (m, 1H), 4.57 (ddd, J = 8.3, 4.0, 3.2 Hz, 1H), 4.34 (dd, J = 9.1, 8.3 Hz, 1H), 4.27 (dd, J = 9.1, 3.2 Hz, 1H), 2.46 (pt, J = 7.0, 3.5 Hz, 1H), 0.94 (dd, J = 17.4, 7.0 Hz, 6H); LRMS (ESI): 344.10 [M + H]⁺.

### Example 65 (S, E) -4-isopropyl-3- (3- (2-trifluoromethoxyphenyl) acryloyl) oxazolidin-2-one

Replace (E) -3- (o-methylphenyl) acrylic acid with (E) -3- (2-trifluoromethoxyphenyl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (2-trifluoromethoxyphenyl) acryloyl) oxazolidin-2-one. ¹H NMR (400 MHz, Chloroform-d) δ 8.11 (d, J = 15.9 Hz, 1H), 7.99 (d, J = 15.8 Hz, 1H), 7.81 (dd, J = 7.8, 1.7 Hz, 1H), 7.44 (ddd, J = 8.2, 7.4, 1.7 Hz, 1H), 7.36 - 7.28 (m, 2H), 4.57 (ddd, J = 8.2, 3.9, 3.1 Hz, 1H), 4.34 (dd, J = 9.1, 8.3 Hz, 1H), 4.27 (dd, J = 9.1, 3.1 Hz, 1H), 2.48 (heptd, J = 7.0, 3.9 Hz, 1H), 0.97 (d, J = 7.0 Hz, 3H), 0.93 (d, J = 7.0 Hz, 3H); LRMS (ESI): 344.10 [M + H]⁺.

### Example 66 (S, E) -4-isopropyl-3- (3- (4-trifluoromethoxyphenyl) acryloyl) oxazolidin-2-one

Replace (E) -3- (o-methylphenyl) acrylic acid with (E) -3- (4-trifluoromethoxyphenyl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (4-trifluoromethoxyphenyl) acryloyl) oxazolidin-2-one. LRMS (ESI): 344.10 [M + H]⁺.

### Example 67 (S, E) -3- (3- ([1,1 '- biphenyl] -3-yl) acryloyl) -4-isopropyloxazolidin-2-one

Replace (E) -3- (o-methylphenyl) acrylic acid with (E) -3- ([1,1 '- biphenyl] -3-yl) acrylic acid, and replace oxazolidin-2-one with (S) -4-isopropyloxazolidin-2-one. The remaining required raw materials, reagents, and preparation methods were the same as in Example 1 to obtain (S, E) -3- (3- ([1,1' - biphenyl] -3-yl) acryloyl) -4-isopropyloxazolidin-2-one. LRMS (ESI): 336.15 [M + H]⁺.

### Example 68 (S, E) -3- (3- (4-fluoro-2- (trifluoromethyl) phenyl) acryloyl) -4-isopropyloxazolidin-2-one

Replace (E) -3- (o-methylphenyl) acrylic acid with (E) -3- (4-fluoro-2-(trifluoromethyl) phenyl) acrylic acid, and replace oxazolidin-2-one with (S) -4-isopropyloxazolidin-2-one. The remaining required raw materials, reagents, and preparation methods were the same as in Example 1 to obtain (S, E) -3- (3- (4-fluoro-2- (trifluoromethyl) phenyl) acryloyl) -4-isopropyloxazolidin-2-one. ¹H NMR (400 MHz, Chloroform-d) δ 8.14 (d, J = 15.5 Hz, 1H), 7.88 (d, J = 14.7 Hz, 2H), 7.60 - 7.36 (m, 1H), 7.30 (d, J = 8.5 Hz, 1H), 4.57 (dd, J = 8.0, 3.9 Hz, 1H), 4.40 - 4.18 (m, 2H), 2.64 - 2.34 (m, 1H), 0.95 (dd, J = 16.0, 7.0 Hz, 8H); LRMS (ESI): 346.10 [M + H]⁺.

### Example 69 (S, E) -3- (3- (benzofuran-2-yl) acryloyl) -4-isopropyloxazolidin-2-one

Replace (E) -3- (o-methylphenyl) acrylic acid with (E) -3- (benzofuran-2-yl) acrylic acid, and replace oxazolin-2-one with (S) -4-isopropyloxazolin-2-one. The remaining required raw materials, reagents, and preparation methods were the same as in Example 1 to obtain (S, E) -3- (3- (benzofuran-2-yl) acryloyl) -4-isopropyloxazolin-2-one. ¹H NMR (400 MHz, Methylene Chloride-d₂) δ 7.96 (dd, J = 15.4, 0.6 Hz, 1H), 7.70 (d, J = 15.4 Hz, 1H), 7.62 (ddd, J = 7.8, 1.3, 0.7 Hz, 1H), 7.54 (dq, J = 8.4, 0.9 Hz, 1H), 7.42 - 7.36 (m, 1H), 7.26 (ddd, J = 8.1, 7.2, 1.0 Hz, 1H), 7.05 (s, 1H), 4.55 (ddd, J = 8.2, 4.0, 3.2 Hz, 1H), 4.33 (dd, J = 9.1, 8.2 Hz, 1H), 4.26 (dd, J = 9.1, 3.2 Hz, 1H), 2.00 (s, 1H), 0.95 (d, J = 7.0 Hz, 3H), 0.91 (d, J = 6.9 Hz, 3H); LRMS (ESI): 300.12 [M + H]⁺.

### Example 70 (S, E) -3- (3- (benzo [b] thiophen-2-yl) acryloyl) -4-isopropyloxazolidin-2-one

Replace (E) -3- (o-methylphenyl) acrylic acid with (E) -3- (benzo [b] thiophen-2-yl) acrylic acid, and replace oxazolidin-2-one with (S) -4-isopropyloxazolidin-2-one. The remaining required raw materials, reagents, and preparation methods were the same as in Example 1 to obtain (S, E) -3- (3- (benzo [b] thiophen-2-yl) acryloyl) -4-isopropyloxazolidin-2-one. ¹H NMR (400 MHz, DMSO-d₆) δ 8.09 - 8.03 (m, 1H), 8.02 - 7.98 (m, 1H), 7.92 - 7.87 (m, 2H), 7.69 (d, J = 15.4 Hz, 1H), 7.44 (pd, J = 7.1, 1.4 Hz, 2H), 4.49 (dt, J = 7.4, 3.7 Hz, 1H), 4.41 - 4.33 (m, 2H), 1.32 - 1.19 (m, 1H), 0.86 (dd, J = 26.2, 6.9 Hz, 6H); LRMS (ESI): 316.09 [M + H]⁺.

### Example 71 (S, E) -4-isopropyl-3- (3- (naphthalen-1-yl) acryloyl) oxazolidin-2-one

Replace (E) -3- (o-methylphenyl) acrylic acid with (E) -3- (naphthalen-1-yl) acrylic acid, and replace oxazolidin-2-one with (S) -4-isopropyloxazolidin-2-one. The remaining required raw materials, reagents, and preparation methods were the same as in Example 1 to obtain (S, E) -4-isopropyl-3- (3- (naphthalen-1-yl) acryloyl) oxazolidin-2-one. ¹H NMR (400 MHz, Chloroform-d) δ 8.74 (d, J = 15.5 Hz, 1H), 8.28 (d, J = 8.4 Hz, 1H), 8.06 (d, J = 15.5 Hz, 1H), 7.95 (dd, J = 7.7, 3.5 Hz, 2H), 7.91 (dd, J = 7.7, 1.3 Hz, 1H), 7.61 (ddd, J = 8.5, 6.8, 1.5 Hz, 1H), 7.58 - 7.51 (m, 2H), 4.63 (dt, J = 8.3, 3.5 Hz, 1H), 4.37 (t, J = 8.7 Hz, 1H), 4.30 (dd, J = 9.1, 3.1 Hz, 1H), 2.54 (pd, J = 7.0, 4.0 Hz, 1H), 1.01 (d, J = 7.0 Hz, 3H), 0.98 (d, J = 6.9 Hz, 3H); LRMS (ESI): 310.10 [M + H]⁺.

### Example 72 (S, E) -3- (3- (benzo [d] [1,3] dioxolan-5-yl) acryloyl) -4-isopropyloxazolidin-2-one

Replace (E) -3- (o-methylphenyl) acrylic acid with (E) -3- (benzo [d] [1,3] dioxolan-5-yl) acrylic acid, and replace oxazolidin-2-one with (S) -4-isopropyloxazolidin-2-one. The remaining required raw materials, reagents, and preparation methods were the same as in Example 1 to obtain (S, E) -3- (3- (benzo [d] [1,3] dioxolan-5-yl) acryloyl) -4-isopropyloxazolidin-2-one. ¹H NMR (400 MHz, Chloroform-d) δ 7.77 (d, J = 0.9 Hz, 2H), 7.15 (d, J = 1.7 Hz, 1H), 7.10 (dd, J = 8.0, 1.7 Hz, 1H), 6.82 (d, J = 8.0 Hz, 1H), 6.02 (s, 2H), 4.56 (ddd, J = 8.2, 4.0, 3.1 Hz, 1H), 4.31 (dd, J = 9.1, 8.3 Hz, 1H), 4.25 (dd, J = 9.0, 3.2 Hz, 1H), 2.46 (pd, J = 7.0, 3.9 Hz, 1H), 0.96 (d, J = 7.0 Hz, 3H), 0.91 (d, J = 6.9 Hz, 3H); LRMS (ESI): 304.11 [M + H]⁺.

### Example 73 (E) -3- (3- (benzo [d] [1,3] dioxolan-5-yl) acryloyl) -4,4-dimethyloxazolidin-2-one

Replace (E) -3- (o-methylphenyl) acrylic acid with (E) -3- (benzo [d] [1,3] dioxolan-5-yl) acrylic acid, and replace oxazolidin-2-one with 4,4-dimethyloxazolidin-2-one. The remaining required raw materials, reagents, and preparation methods were the same as in Example 1 to obtain (E) -3- (3- (benzo [d] [1,3] dioxolan-5-yl) acryloyl) -4,4-dimethyloxazolidin-2-one. ¹H NMR (400 MHz, Chloroform-d) δ 7.71 (d, J = 15.6 Hz, 1H), 7.56 (d, J = 15.5 Hz, 1H), 7.12 (d, J = 1.7 Hz, 1H), 7.07 (dd, J = 8.0, 1.7 Hz, 1H), 6.81 (d, J = 8.0 Hz, 1H), 6.01 (s, 2H), 4.05 (s, 2H), 1.64 (s, 6H); LRMS (ESI): 290.10 [M + H]⁺.

### Example 74 (E) -4,4-dimethyl-3- (3- (2- (trifluoromethyl) phenyl) acryloyl) oxazolidin-2-one

Replace (E) -3- (o-methylphenyl) acrylic acid with (E) -3- (2-trifluoromethylphenyl) acrylic acid, and replace oxazolidin-2-one with 4,4-dimethyloxazolidin-2-one. The remaining required raw materials, reagents, and preparation methods were the same as in Example 1 to obtain (E) -4,4-dimethyl-3- (3- (2- (trifluoromethylphenyl) acryloyl) oxazolidin-2-one. ¹H NMR (400 MHz, Chloroform-d) δ 8.15 (d, J = 15.4 Hz, 1H), 7.83 (d, J = 7.8 Hz, 1H), 7.79 - 7.65 (m, 2H), 7.53 (dt, J = 37.3, 7.7 Hz, 2H), 4.08 (s, 2H), 1.66 (s, 6H); LRMS (ESI): 314.09 [M + H]⁺.

### Example 75 (E) -4,4-dimethyl-3- (3- (3- (trifluoromethoxy) phenyl) acryloyl) oxazolidin-2-one

Replace (E) -3- (o-methylphenyl) acrylic acid with (E) -3- (3-trifluoromethoxyphenyl) acrylic acid, and replace oxazolidin-2-one with 4,4-dimethyloxazolidin-2-one. The remaining required raw materials, reagents, and preparation methods were the same as in Example 1 to obtain (E) -4,4-dimethyl-3- (3- (trifluoromethoxyphenyl) acryloyl) oxazolidin-2-one. ¹H NMR (400 MHz, Chloroform-d) δ 7.73 (s, 2H), 7.53 (dt, J = 7.7, 1.2 Hz, 1H), 7.45 - 7.38 (m, 2H), 7.25 - 7.22 (m, 1H), 4.08 (s, 2H), 1.65 (s, 6H); LRMS (ESI): 330.09 [M + H]⁺.

### Example 76 (E) -4,4-dimethyl-3- (3- (2- (trifluoromethoxy) phenyl) acryloyl) oxazolidin-2-one

Replace (E) -3- (o-methylphenyl) acrylic acid with (E) -3- (2-trifluoromethoxyphenyl) acrylic acid, and replace oxazolidin-2-one with 4,4-dimethyloxazolidin-2-one. The remaining required raw materials, reagents, and preparation methods were the same as in Example 1 to obtain (E) -4,4-dimethyl-3- (3- (2- (trifluoromethoxyphenyl) acryloyl) oxazolidin-2-one. ¹H NMR (400 MHz, Chloroform-d) δ 8.04 (d, J = 15.8 Hz, 1H), 7.88 - 7.66 (m, 2H), 7.47 - 7.38 (m, 1H), 7.38 - 7.20 (m, 2H), 4.08 (s, 2H), 1.66 (s, 6H); LRMS (ESI): 330.09 [M + H]⁺.

### Example 77 (E) -3- (3- (4-fluoro-2- (trifluoromethyl) phenyl) acryloyl) -4,4-dimethyloxazolidin-2-one

Replace (E) -3- (o-methylphenyl) acrylic acid with (E) -3- (4-fluoro-2-trifluoromethylphenyl) acrylic acid, and replace oxazolidin-2-one with 4,4-dimethyloxazolidin-2-one. The remaining required raw materials, reagents, and preparation methods were the same as in Example 1 to obtain (E) -3- (3- (4-fluoro-2-(trifluoromethylphenyl) acryloyl) -4,4-dimethyloxazolidin-2-one. ¹H NMR (400 MHz, Chloroform-d) δ 8.07 (d, J = 15.6 Hz, 1H), 7.84 (dd, J = 8.8, 5.4 Hz, 1H), 7.67 (d, J = 15.5 Hz, 1H), 7.45 - 7.38 (m, 1H), 7.27 (d, J = 6.5 Hz, 1H), 4.08 (s, 2H), 1.66 (s, 6H); LRMS (ESI): 332.12 [M + H]⁺.

### Example 78 (E) -3- (3- ([1,1 '- biphenyl] -3-yl) acryloyl) thiazolidin-2-one

Replace (E) -3- (o-methylphenyl) acrylic acid with (E) -3- ([1,1 '- biphenyl] -3-yl) acrylic acid, and replace oxazolidin-2-one with thiazolidin-2-one. The remaining required raw materials, reagents, and preparation methods were the same as in Example 1 to obtain (E) -3- (3- ([1,1' - biphenyl] -3-yl) acryloyl) thiazolidin-2-one (yield 89.0%). ¹H NMR (400 MHz, DMSO-d₆) δ 7.81 (d, J = 15.0 Hz, 1H), 7.73 (dd, J = 7.5, 2.0 Hz, 2H), 7.66 - 7.44 (m, 7H), 7.44 - 7.34 (m, 1H), 3.70 (t, J = 6.1 Hz, 2H), 3.55 (t, J = 6.0 Hz, 2H). LRMS (ESI): 310.08 [M+H]+.

### Example 79 (E) -3- (3- (2-phenoxyphenyl) acryloyl) thiazolidin-2-one

Replace (E) -3- (2-phenoxyphenyl) acrylic acid with (E) -3- (2-phenoxyphenyl) acrylic acid, and replace oxazolidin-2-one with thiazolidin-2-one. The remaining required raw materials, reagents, and preparation methods were the same as in Example 1 to obtain (E) - 3- (3- (2-phenoxyphenyl) acryloyl) thiazolidin-2-one (yield 89.6%). ¹H NMR (400 MHz, DMSO-d₆) δ 7.94 (dd, J = 15.0, 0.9 Hz, 1H), 7.68 - 7.60 (m, 2H), 7.46 (td, J = 7.5, 2.0 Hz, 1H), 7.44 - 7.34 (m, 2H), 7.23 - 7.09 (m, 2H), 7.12 - 7.01 (m, 3H), 3.70 (t, J = 6.1 Hz, 2H), 3.55 (t, J = 6.0 Hz, 2H). LRMS (ESI): 326.08 [M+H]+.

### Example 80 (E) -3- (3- (2-trifluoromethylphenyl) acryloyl) thiazolidine-2-one

Replace (E) -3- (o-methylphenyl) acrylic acid with (E) -3- (2-trifluoromethylphenyl) acrylic acid, replace oxazolidin-2-one with thiazolidin-2-one. The remaining required raw materials, reagents, and preparation methods were the same as in Example 1 to obtain (E) - 3- (3- (2-trifluoromethylphenyl) acryloyl) thiazolidin-2-one (yield 90.6%). ¹H NMR (400 MHz, DMSO-d₆) δ 7.86 - 7.79 (m, 1H), 7.76 (td, J = 7.5, 2.0 Hz, 1H), 7.69 - 7.55 (m, 2H), 7.47 (td, J = 7.4, 2.1 Hz, 1H), 7.30 - 7.23 (m, 1H), 3.70 (t, J = 6.2 Hz, 2H), 3.55 (t, J = 6.2 Hz, 2H). LRMS (ESI): 302.04 [M+H]⁺.

### Example 81 (E) -3- (3- (thiphen-2-yl) acryloyl) thiazolidin-2-one

Replace (E) -3- (o-methylphenyl) acrylic acid with (E) -3- (thiphen-2-yl) acrylic acid, replace oxazolidin-2-one with thiazolidin-2-one. The remaining required raw materials, reagents, and preparation methods were the same as in Example 1 to obtain (E) -3- (3-(thiphen-2-yl) acryloyl) thiazolidin-2-one (yield 85.1%). ¹H NMR (400 MHz, DMSO-d₆) δ 7.86 - 7.75 (m, 2H), 7.70 (dd, J = 7.4, 1.6 Hz, 1H), 7.60 (d, J = 15.0 Hz, 1H), 7.20 (t, J = 7.4 Hz, 1H), 3.70 (t, J = 6.1 Hz, 2H), 3.55 (t, J = 6.0 Hz, 2H). LRMS (ESI): 240.01 [M+H]⁺.

### Example 83 (S, E) -4-phenyl-3- (3- (2-trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolin-2-one with (S) -4-phenyloxazolin-2-one-5,5-d2. The remaining required raw materials, reagents, and preparation methods were the same as in Example 1 to obtain (S, E) -4-phenyl-3- (3- (2-trifluoromethylphenyl) acryloyl) oxazolin-2-one-5,5-d2. ¹H NMR (400 MHz, DMSO-d₆) δ 7.94 (d, J = 7.8 Hz, 1H), 7.89 - 7.77 (m, 4H), 7.67 (t, J = 7.6 Hz, 1H), 7.44 - 7.30 (m, 5H), 5.57 (s, 1H). LRMS (ESI): 364.11 [M+H]⁺.

### Example 84 (S, E) -4-phenyl-3- (3- (2-trifluoromethoxyphenyl) acryloyl) oxazolidin-2-one-5,5-d₂

Replace (E) -3- (o-methylphenyl) acrylic acid with (E) -3- (2-trifluoromethoxyphenyl) acrylic acid, and replace oxazolin-2-one with (S) -4-phenyloxazolin-2-one-5,5-d2. The remaining required raw materials, reagents, and preparation methods were the same as in Example 1 to obtain (S, E) -4-phenyl-3- (3- (2-trifluoromethoxyphenyl) acryloyl) oxazolin-2-one-5,5-d2. ¹H NMR (400 MHz, DMSO-d₆) δ 7.95 (d, J = 15.0 Hz, 1H), 7.70 - 7.60 (m, 2H), 7.39 - 7.28 (m, 4H), 7.28 - 7.18 (m, 2H), 7.02 (t, J = 7.1 Hz, 2H), 5.50 (s, 1H). LRMS (ESI): 380.11 [M+H]⁺.

### Example 85 (S, E) -4-phenyl-3- (3- (2-phenoxyphenyl) acryloyl) oxazolidin-2-one-5,5-d₂

Replace (E) -3- (o-methylphenyl) acrylic acid with (E) -3- (2-phenoxyphenyl) acrylic acid, and replace oxazolin-2-one with (S) -4-phenyloxazolin-2-one-5,5-d2. The remaining required raw materials, reagents, and preparation methods were the same as in Example 1 to obtain (S, E) -4-phenyl-3- (3- (2-phenoxyphenyl) acryloyl) oxazolin-2-one-5,5-d2. ¹H NMR (400 MHz, DMSO-d₆) δ 7.95 (d, J = 15.0 Hz, 1H), 7.68 - 7.58 (m, 2H), 7.46 (td, J = 7.4, 2.0 Hz, 1H), 7.42 - 7.19 (m, 7H), 7.19 - 7.09 (m, 1H), 7.09 - 7.01 (m, 2H), 5.50 (s, 1H). LRMS (ESI): 388.14 [M+H]⁺.

### Example 86 (S, E) -3- (3- ([1,1 '- biphenyl] -3-yl) acryloyl) -4-phenyloxazolidin-2-one-5,5-d₂

Replace (E) -3- (o-methylphenyl) acrylic acid with (E) -3- ([1,1 '- biphenyl] -3-yl) acrylic acid, and replace oxazolidin-2-one with (S) -4-isopropyloxazolidin-2-one. The remaining required raw materials, reagents, and preparation methods were the same as in Example 1 to obtain (S, E) -3- (3- ([1,1' - biphenyl] -3-yl) acryloyl) -4-phenyloxazolidin-2-one-5,5-d2. ¹H NMR (400 MHz, DMSO-d₆) δ 7.81 (d, J = 15.0 Hz, 1H), 7.73 (dd, J = 7.6, 2.0 Hz, 3H), 7.62 (dt, J = 7.6, 2.1 Hz, 1H), 7.54 - 7.47 (m, 3H), 7.47 - 7.32 (m, 7H), 7.32 - 7.25 (m, 1H), 7.24 (ddt, J = 5.3, 3.9, 1.6 Hz, 1H), 5.50 (s, 1H). LRMS (ESI): 372.15 [M + H]⁺

### Example 87 (S, E) -4-phenyl-3- (3- (3-phenoxyphenyl) acryloyl) oxazolidin-2-one-5,5-d₂

Replace (E) -3- (o-methylphenyl) acrylic acid with (E) -3- (3-phenoxyphenyl) acrylic acid, and replace oxazolin-2-one with (S) -4-phenyloxazolin-2-one-5,5-d2. The remaining required raw materials, reagents, and preparation methods were the same as in Example 1 to obtain (S, E) -4-phenyl-3- (3- (3-phenoxyphenyl) acryloyl) oxazolin-2-one-5,5-d2. ¹H NMR (400 MHz, DMSO-d₆) δ 7.81 (d, J = 15.1 Hz, 1H), 7.44 - 7.29 (m, 9H), 7.29 - 7.20 (m, 2H), 7.14 (tt, J = 7.4, 2.0 Hz, 1H), 7.05 (dd, J = 7.5, 2.0 Hz, 2H), 6.88 (dp, J = 4.1, 2.1 Hz, 2H), 5.50 (s, 1H). LRMS (ESI): 388.14 [M+H]⁺.

### Example 88 (E) -4-phenyl-3- (3- (2-trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolin-2-one with 4-phenyloxazolin-2-one-5,5-d2. The remaining required raw materials, reagents, and preparation methods were the same as in Example 1 to obtain (E) -4-phenyl-3- (3- (2-trifluoromethylphenyl) acryloyl) oxazolin-2-one-5,5-d2. ¹H NMR (400 MHz, DMSO-d₆) δ 7.94 (d, J = 7.8 Hz, 1H), 7.89 - 7.77 (m, 4H), 7.67 (t, J = 7.6 Hz, 1H), 7.44 - 7.30 (m, 5H), 5.57 (s, 1H). LRMS (ESI): 364.11 [M+H]⁺.

### Example 89 (E) -3- (3- (2-trifluoromethylphenyl) acryloyl) oxazolidin-2-one-4,4,5-d4

Replace (E) -3- (o-methylphenyl) acrylic acid with (E) -3- (2-trifluoromethylphenyl) acrylic acid, and replace oxazolidin-2-one with oxazolidin-2-one-4,4,5-d4. The remaining required raw materials, reagents, and preparation methods were the same as in Example 1 to obtain (E) -3- (3- (2-trifluoromethylphenyl) acryloyl) oxazolidin-2-one-4,4,5-d4. ¹H NMR (400 MHz, DMSO-d₆) δ 8.00 - 7.89 (m, 2H), 7.76 (td, J = 7.5, 2.1 Hz, 1H), 7.64 (d, J = 15.0 Hz, 1H), 7.35 (td, J = 7.5, 2.0 Hz, 1H), 7.27 - 7.19 (m, 1H). LRMS (ESI): 290.09 [M+H]⁺.

### Example 90 (E) -3- (3- (2-methoxyphenyl) acryloyl) oxazolidin-2-one-4,4,5-d4

Replace (E) -3- (o-methoxyphenyl) acrylic acid with (E) -3- (2-methoxyphenyl) acrylic acid, and replace oxazolidin-2-one with oxazolidin-2-one-4,4,5-d4. The remaining required raw materials, reagents, and preparation methods were the same as in Example 1 to obtain (E) -3- (3- (2-methoxyphenyl) acryloyl) oxazolidin-2-one-4,4,5-d4. ¹H NMR (400 MHz, DMSO-d₆) δ 7.95 (dd, J = 15.0, 1.0 Hz, 1H), 7.70 - 7.60 (m, 2H), 7.49 (td, J = 7.5, 2.0 Hz, 1H), 7.11 - 6.97 (m, 2H), 3.91 (s, 3H). LRMS (ESI): 252.11 [M+H]⁺.

### Example 91 (E) -3- (3- (3-methoxyphenyl) acryloyl) oxazolidin-2-one-4,4,5-d4

Replace (E) -3- (o-methoxyphenyl) acrylic acid with (E) -3- (3-methoxyphenyl) acrylic acid, and replace oxazolidin-2-one with oxazolidin-2-one-4,4,5-d4. The remaining required raw materials, reagents, and preparation methods were the same as in Example 1 to obtain (E) -3- (3- (3-methoxyphenyl) acryloyl) oxazolidin-2-one-4,4,5-d4. ¹H NMR (400 MHz, DMSO-d₆) δ 7.81 (d, J = 15.0 Hz, 1H), 7.41 - 7.27 (m, 3H), 7.04 (t, J = 1.6 Hz, 1H), 6.86 - 6.77 (m, 1H), 3.73 (s, 3H). LRMS (ESI): 252.11 [M+H]⁺.

### Example 92 (E) -3- (3- (4-methoxyphenyl) acryloyl) oxazolidin-2-one-4,4,5-d4

Replace (E) -3- (o-methoxyphenyl) acrylic acid with (E) -3- (4-methoxyphenyl) acrylic acid, and replace oxazolidin-2-one with oxazolidin-2-one-4,4,5-d4. The remaining required raw materials, reagents, and preparation methods were the same as in Example 1 to obtain (E) -3- (3- (4-methoxyphenyl) acryloyl) oxazolidin-2-one-4,4,5-d4. ¹H NMR (400 MHz, DMSO-d₆) δ 7.81 (d, J = 15.0 Hz, 1H), 7.72 - 7.64 (m, 2H), 7.37 (dt, J = 15.2, 1.0 Hz, 1H), 7.10 - 7.02 (m, 2H), 3.79 (s, 3H). LRMS (ESI): 252.11 [M+H]⁺.

### Example 93 (E) -3- (3- ([1,1 '- biphenyl] -3-yl) acryloyl) oxazolidin-2-one-4,4,5-d4

Replace (E) -3- (o-methylphenyl) acrylic acid with (E) -3- ([1,1 '- biphenyl] -3-yl) acrylic acid, and replace oxazolidin-2-one with oxazolidin-2-one-4,4,5-d4. The remaining required raw materials, reagents, and preparation methods were the same as in Example 1 to obtain (E) -3- (3- ([1,1' - biphenyl] -3-yl) acryloyl) oxazolidin-2-one-4,4,5-d4. ¹H NMR (400 MHz, DMSO-d₆) δ 7.81 (d, J = 15.0 Hz, 1H), 7.73 (dd, J = 7.6, 2.0 Hz, 3H), 7.62 (dt, J = 7.6, 2.1 Hz, 1H), 7.54 - 7.43 (m, 4H), 7.43 - 7.32 (m, 2H). LRMS (ESI): 298.13 [M+H]⁺.

### Example 94 (E) -3- (3- (3-phenoxyphenyl) acryloyl) oxazolidin-2-one-4,4,5-d4

Replace (E) -3- (o-methylphenyl) acrylic acid with (E) -3- (3-phenoxyphenyl) acrylic acid, and replace oxazolidin-2-one with oxazolidin-2-one-4,4,5-d4. The remaining required raw materials, reagents, and preparation methods were the same as in Example 1 to obtain (E) -3- (3- (3-phenoxyphenyl) acryloyl) oxazolidin-2-one-4,4,5-d4. ¹H NMR (400 MHz, DMSO-d₆) δ 7.81 (d, J = 15.1 Hz, 1H), 7.44 - 7.31 (m, 4H), 7.29 (t, J = 7.7 Hz, 1H), 7.14 (tt, J = 7.5, 2.0 Hz, 1H), 7.09 - 7.01 (m, 2H), 6.88 (dp, J = 4.1, 2.1 Hz, 2H). LRMS (ESI): 314.13 [M+H]⁺.

### Example 95 (E) -3- (3- (4-methoxyphenyl) acryloyl) oxazolidin-2-one-4,4-d2

Replace (E) -3- (o-methylphenyl) acrylic acid with (E) -3- (4-methoxyphenyl) acrylic acid, and replace oxazolidin-2-one with oxazolidin-2-one-4,4-d2. The remaining required raw materials, reagents, and preparation methods were the same as in Example 1 to obtain (E) -3- (3- (4-methoxyphenyl) acryloyl) oxazolidin-2-one-4,4-d2. ¹H NMR (400 MHz, DMSO-d₆) δ 7.81 (d, J = 15.0 Hz, 1H), 7.72 - 7.64 (m, 2H), 7.37 (dt, J = 15.2, 1.0 Hz, 1H), 7.10 - 7.02 (m, 2H), 4.30 (s, 2H), 3.79 (s, 3H). LRMS (ESI): 250.10 [M+H]⁺.

### Example 96 (E) -3- (3- (4-methoxyphenyl) acryloyl) oxazolidin-2-one-5,5-d₂

Replace (E) -3- (o-methylphenyl) acrylic acid with (E) -3- (4-methoxyphenyl) acrylic acid, and replace oxazolidin-2-one with oxazolidin-2-one-5,5-d2. The remaining required raw materials, reagents, and preparation methods were the same as in Example 1 to obtain (E) -3- (3- (4-methoxyphenyl) acryloyl) oxazolidin-2-one-5,5-d2. ¹H NMR (400 MHz, DMSO-d₆) δ 7.81 (d, J = 15.0 Hz, 1H), 7.72 - 7.64 (m, 2H), 7.37 (dt, J = 15.1, 1.0 Hz, 1H), 7.10 - 7.02 (m, 2H), 3.80 (s, 2H), 3.79 (s, 3H). LRMS (ESI): 250.10 [M+H]⁺.

### Example 97 (E) -3- (3- (3-phenoxyphenyl) acryloyl) oxazolidin-2-one-4,4-d2

Replace (E) -3- (o-methylphenyl) acrylic acid with (E) -3- (3-phenoxyphenyl) acrylic acid, and replace oxazolidin-2-one with oxazolidin-2-one-4,4-d2. The remaining required raw materials, reagents, and preparation methods were the same as in Example 1 to obtain (E) -3- (3- (3-phenoxyphenyl) acryloyl) oxazolidin-2-one-4,4-d2. ¹H NMR (400 MHz, DMSO-d₆) δ 7.81 (d, J = 15.1 Hz, 1H), 7.44 - 7.31 (m, 4H), 7.29 (t, J = 7.7 Hz, 1H), 7.14 (tt, J = 7.5, 2.0 Hz, 1H), 7.09 - 7.01 (m, 2H), 6.88 (dp, J = 5.7, 2.1 Hz, 2H), 4.30 (s, 2H). LRMS (ESI): 312.12 [M+H]⁺.

### Example 98 (E) -3- (3- (3-phenoxyphenyl) acryloyl) oxazolidin-2-one-5,5-d₂

Replace (E) -3- (o-methylphenyl) acrylic acid with (E) -3- (3-phenoxyphenyl) acrylic acid, and replace oxazolidin-2-one with oxazolidin-2-one-5,5-d2. The remaining required raw materials, reagents, and preparation methods were the same as in Example 1 to obtain (E) -3- (3- (3-phenoxyphenyl) acryloyl) oxazolidin-2-one-5,5-d2. ¹H NMR (400 MHz, DMSO-d₆) δ 7.81 (d, J = 15.1 Hz, 1H), 7.44 - 7.31 (m, 4H), 7.29 (t, J = 7.7 Hz, 1H), 7.14 (tt, J = 7.5, 2.0 Hz, 1H), 7.09 - 7.01 (m, 2H), 6.88 (dp, J = 5.7, 2.1 Hz, 2H), 3.80 (s, 2H). LRMS (ESI): 312.12 [M+H]⁺.

### Example 99 (E) -3- (3- (2-trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolidin-2-one with oxazolidin-2-one-5,5-d2. The remaining required raw materials, reagents, and preparation methods were the same as in Example 1 to obtain (E) -3- (3- (2-trifluoromethylphenyl) acryloyl) oxazolidin-2-one-5,5-d2. ¹H NMR (400 MHz, DMSO-d₆) δ 8.00 - 7.89 (m, 2H), 7.76 (td, J = 7.5, 2.1 Hz, 1H), 7.64 (d, J = 15.0 Hz, 1H), 7.35 (td, J = 7.5, 2.0 Hz, 1H), 7.23 (ddd, J = 7.6, 2.1, 1.0 Hz, 1H), 3.80 (s, 2H). LRMS (ESI): 288.07 [M+H]⁺.

### Example 100 (E) -3- (3- ([1,1 '- biphenyl] -3-yl) acryloyl) oxazolidin-2-one-5,5-d₂

Replace (E) -3- (o-methylphenyl) acrylic acid with (E) -3- ([1,1 '- biphenyl] -3-yl) acrylic acid, and replace oxazolidin-2-one with oxazolidin-2-one-5,5-di. The remaining required raw materials, reagents, and preparation methods were the same as in Example 1 to obtain (E) -3- (3- ([1,1' - biphenyl] -3-yl) acryloyl) oxazolidin-2-one-5,5-di. ¹H NMR (400 MHz, DMSO-d₆) δ 7.81 (d, J = 15.0 Hz, 1H), 7.73 (dq, J = 8.3, 2.1 Hz, 3H), 7.62 (dt, J = 7.6, 2.1 Hz, 1H), 7.54 - 7.43 (m, 4H), 7.39 (s, 1H), 7.44 - 7.32 (m, 1H), 3.80 (s, 2H). LRMS (ESI): 296.12 [M+H]⁺.

### Example 101 (E) -4,4-dimethyl-3- (3- (2- (trifluoromethyl) phenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolidin-2-one with 4,4-dimethyloxazolidin-2-one-5,5-***d₂***. The remaining required raw materials, reagents, and preparation methods were the same as in Example 1 to obtain (E) -4,4-dimethyl-3- (3- (2- (trifluoromethylphenyl) acryloyl) oxazolidin-2-one-5,5-d2. ¹H NMR (400 MHz, DMSO-d₆) δ 8.00 - 7.89 (m, 2H), 7.76 (td, J = 7.5, 2.0 Hz, 1H), 7.64 (d, J = 15.0 Hz, 1H), 7.35 (td, J = 7.5, 2.1 Hz, 1H), 7.23 (ddd, J = 7.5, 2.1, 1.0 Hz, 1H), 1.29 (s, 6H). LRMS (ESI): 316.12 [M+H]⁺.

### Example 102 (E) -4- (4 '- fluorophenyl) -3- (3- (2- (trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolin-2-one with 4- (4 '- fluorophenyl) oxazolin-2-one-5,5-d2. The remaining required raw materials, reagents, and preparation methods were the same as in Example 1 to obtain (E) -4- (4' - fluorophenyl) -3- (3- (2- (trifluoromethylphenyl) acryloyl) oxazolin-2-one-5,5-d2. ¹H NMR (400 MHz, DMSO-d₆) δ 8.00 - 7.89 (m, 2H), 7.76 (td, J = 7.5, 2.1 Hz, 1H), 7.64 (d, J = 15.0 Hz, 1H), 7.35 (td, J = 7.5, 2.1 Hz, 1H), 7.27 - 7.12 (m, 5H), 5.50 (s, 1H). LRMS (ESI): 382.10 [M + H]⁺.

### Example 103 (S, E) -4- (4 '- fluorophenyl) -3- (3- (2- (trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolin-2-one with (S) -4- (4 '- fluorophenyl) oxazolin-2-one-5,5-d2. The remaining required raw materials, reagents, and preparation methods were the same as in Example 1 to obtain (S, E) -4- (4' - fluorophenyl) -3- (3- (2-(trifluoromethylphenyl) acryloyl) oxazolin-2-one-5,5-d2. ¹H NMR (400 MHz, DMSO-d₆) δ 8.00 - 7.89 (m, 2H), 7.76 (td, J = 7.5, 2.1 Hz, 1H), 7.64 (d, J = 15.0 Hz, 1H), 7.35 (td, J = 7.5, 2.1 Hz, 1H), 7.27 - 7.12 (m, 5H), 5.50 (s, 1H). LRMS (ESI): 382.10 [M + H]⁺.

### Example 104 (E) -4-benzyl-3- (3- (2-trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolin-2-one with 4-benzyloxazolin-2-one-5,5-d₂. The remaining required raw materials, reagents, and preparation methods were the same as in Example 1 to obtain (E) -4-benzyl-3- (3- (2-trifluoromethylphenyl) acryloyl) oxazolin-2-one-5,5-d₂. ¹H NMR (400 MHz, DMSO-d₆) δ 8.00 - 7.89 (m, 2H), 7.76 (td, J = 7.5, 2.1 Hz, 1H), 7.64 (d, J = 15.0 Hz, 1H), 7.35 (td, J = 7.5, 2.1 Hz, 1H), 7.33 - 7.14 (m, 6H), 4.60 (t, J = 5.3 Hz, 1H), 3.04 (dd, J = 12.4, 5.3 Hz, 1H), 2.78 (dd, J = 12.4, 5.3 Hz, 1H). LRMS (ESI): 378.12 [M + H]⁺.

### Example 105 (S, E) -4-benzyl-3- (3- (2-trifluoromethylphenyl) acryloyl) oxazolidin-2-one-5,5-d₂

Replace (E) -3- (o-methylphenyl) acrylic acid with (E) -3- (2-trifluoromethylphenyl) acrylic acid, and replace oxazolin-2-one with (S) -4-benzyloxazolin-2-one-5,5-d₂. The remaining required raw materials, reagents, and preparation methods were the same as in Example 1 to obtain (S, E) -4-benzyl-3- (3- (2-trifluoromethylphenyl) acryloyl) oxazolin-2-one-5,5-d2. ¹H NMR (400 MHz, DMSO-d₆) δ 8.00 - 7.89 (m, 2H), 7.76 (td, J = 7.5, 2.1 Hz, 1H), 7.64 (d, J = 15.0 Hz, 1H), 7.35 (td, J = 7.5, 2.1 Hz, 1H), 7.33 - 7.14 (m, 6H), 4.60 (t, J = 5.3 Hz, 1H), 3.04 (dd, J = 12.4, 5.3 Hz, 1H), 2.78 (dd, J = 12.4, 5.3 Hz, 1H). LRMS (ESI): 378.12 [M + H]⁺.

### Example 106 (S, E) -4- (4 '- fluorophenyl) -3- (3- (2- (trifluoromethoxyphenyl) acryloyl) oxazolidin-2-one-5,5-d₂

Replace (E) -3- (o-methylphenyl) acrylic acid with (E) -3- (2-trifluoromethoxyphenyl) acrylic acid, and replace oxazolin-2-one with (S) -4- (4 '- fluorophenyl) oxazolin-2-one-5,5-d₂. The remaining required raw materials, reagents, and preparation methods were the same as in Example 1 to obtain (S, E) -4- (4' - fluorophenyl) -3- (3- (2-(trifluoromethoxyphenyl) acryloyl) oxazolin-2-one-5,5-d2. ¹H NMR (400 MHz, DMSO-d₆) δ 7.95 (dd, J = 15.0, 0.9 Hz, 1H), 7.70 - 7.60 (m, 2H), 7.28 - 7.12 (m, 5H), 7.02 (t, J = 7.1 Hz, 2H), 5.50 (s, 1H). LRMS (ESI): 398.09 [M + H]⁺.

### Example 107 (S, E) -4- (4 '- fluorophenyl) -3- (3- (2- (phenoxyphenyl) acryloyl) oxazolidin-2-one-5,5-d₂

Replace (E) -3- (o-methylphenyl) acrylic acid with (E) -3- (2-phenoxyphenyl) acrylic acid, replace oxazolidin-2-one with (S) -4- (4 '- fluorophenyl) oxazolidin-2-one-5,5-d2. The remaining required raw materials, reagents, and preparation methods were the same as in Example 1 to obtain (S, E) -4- (4' - fluorophenyl) -3- (3- (2- (phenoxyphenyl) acryloyl) oxazolidin-2-one-5,5-d2. ¹H NMR (400 MHz, DMSO-d₆) δ 7.95 (dd, J = 15.0, 0.9 Hz, 1H), 7.68 - 7.58 (m, 2H), 7.46 (td, J = 7.4, 2.0 Hz, 1H), 7.44 - 7.34 (m, 2H), 7.31 - 7.18 (m, 3H), 7.21 - 7.10 (m, 3H), 7.14 - 7.01 (m, 3H), 5.50 (s, 1H). LRMS (ESI): 406.13 [M + H]⁺.

### Example 108 (S, E) -3- (3- ([1,1 '- biphenyl] -3-yl) acryloyl) -4- (4' - fluorophenyl) oxazolidin-2-one-5,5-d₂

Replace (E) -3- (o-methylphenyl) acrylic acid with (E) -3- ([1,1 '- biphenyl] -3-yl) acrylic acid, and replace oxazolidin-2-one with (S) -4- (4' - fluorophenyl) oxazolidin-2-one-5,5-d₂. The remaining required raw materials, reagents, and preparation methods were the same as in Example 1 to obtain (S, E) -3- (3- ([1,1 '- biphenyl] -3-yl) acryloyl) -4- (4' - fluorophenyl) oxazolidin-2-one-5,5-d2. ¹H NMR (400 MHz, DMSO-d₆) δ 7.81 (d, J = 15.0 Hz, 1H), 7.73 (dd, J = 7.5, 2.0 Hz, 3H), 7.62 (dt, J = 7.6, 2.1 Hz, 1H), 7.54 - 7.43 (m, 4H), 7.44 - 7.33 (m, 2H), 7.26 - 7.12 (m, 4H), 5.50 (s, 1H). LRMS (ESI): 390.13 [M + H]⁺.

### Example 109 (S, E) -4- (4 '- fluorophenyl) -3- (3- (3- (phenoxyphenyl) acryloyl) oxazolidin-2-one-5,5-d₂

Replace (E) -3- (o-methylphenyl) acrylic acid with (E) -3- (3-phenoxyphenyl) acrylic acid, replace oxazolidin-2-one with (S) -4- (4 '- fluorophenyl) oxazolidin-2-one-5,5-d2. The remaining required raw materials, reagents, and preparation methods were the same as in Example 1 to obtain (S, E) -4- (4' - fluorophenyl) -3- (3- (3- (phenoxyphenyl) acryloyl) oxazolidin-2-one-5,5-d2. ¹H NMR (400 MHz, DMSO-d₆) δ 7.81 (d, J = 15.1 Hz, 1H), 7.44 - 7.09 (m, 11H), 7.09 - 7.01 (m, 2H), 6.88 (dp, J = 4.1, 2.1 Hz, 2H), 5.50 (s, 1H). LRMS (ESI): 406.13 [M + H]⁺.

### Example 110 (R, E) -5,5-dimethyl-4-phenyl-3- (3- (2- (trifluoromethyl) phenyl) acryloyl) oxazolidin-2-one

Replace (E) -3- (o-methylphenyl) acrylic acid with (E) -3- (o-trifluoromethylphenyl) acrylic acid, and replace oxazolin-2-one with (R) -5,5-dimethyl-4-phenyloxazolin-2-one. The remaining required raw materials, reagents, and preparation methods were the same as in Example 1 to obtain (R, E) -5,5-dimethyl-4-phenyl-3- (3- (2- (trifluoromethylphenyl) phenyl) acryloyl) oxazolin-2-one (yield 64.2%). ¹H NMR (500 MHz, Chloroform-d) δ 8.16 (dq, J = 15.5, 2.3 Hz, 1H), 8.00 (d, J = 15.6 Hz, 1H), 7.89 (d, J = 7.8 Hz, 1H), 7.69 (d, J = 7.8 Hz, 1H), 7.59 (t, J = 7.6 Hz, 1H), 7.51 - 7.47 (m, 1H), 7.42 - 7.33 (m, 3H), 7.22 - 7.19 (m, 2H), 5.20 (s, 1H), 1.65 (s, 3H), 1.04 (s, 3H). HRMS (ESI): 390.1315 [M+H]⁺.

### Example 111 (S, E) -5,5-dimethyl-4-phenyl-3- (3- (2- (trifluoromethyl) phenyl) acryloyl) oxazolidin-2-one

Replace (E) -3- (o-methylphenyl) acrylic acid with (E) -3- (o-trifluoromethylphenyl) acrylic acid, and replace oxazolin-2-one with (S) -5,5-dimethyl-4-phenyloxazolin-2-one. The remaining required raw materials, reagents, and preparation methods were the same as in Example 1 to obtain (S, E) -5,5-dimethyl-4-phenyl-3- (3- (2- (trifluoromethylphenyl) phenyl) acryloyl) oxazolin-2-one (yield 71.5%). ¹H NMR (500 MHz, Chloroform-d) δ 8.19 (ddd, J = 7.4, 1.5, 0.6 Hz, 1H), 8.02 (td, J = 7.4, 1.6 Hz, 1H), 7.96 (dd, J = 7.5, 1.6 Hz, 1H), 7.90 (td, J = 7.5, 1.6 Hz, 1H), 7.88 - 7.82 (m, 1H), 7.62 (d, J = 15.0 Hz, 1H), 7.36 - 7.24 (m, 5H), 5.25 - 5.21 (m, 1H), 1.46 (d, J = 1.6 Hz, 3H), 1.41 (d, J = 1.5 Hz, 3H). LRMS (ESI): 390.20 [M+H]⁺.

### Example 112 (R, E) -7-phenyl-6- (3- (2- (trifluoromethyl) phenyl) acryloyl) -4-oxa-6-azaspiro [2.4] heptan-5-one

Replace (E) -3- (o-methylphenyl) acrylic acid with (E) -3- (o-trifluoromethylphenyl) acrylic acid, and replace oxazolin-2-one with (R) -7-phenyl-4-oxa-6-azaspiro [2.4] heptan-5-one. The remaining required raw materials, reagents, and preparation methods were the same as in Example 1 to obtain (R, E) -7-phenyl-6- (3- (2- (trifluoromethylphenyl) phenyl) acryloyl) -4-oxa-6-azaspiro [2.4] heptan-5-one (yield 49.2%). ¹H NMR (500 MHz, Chloroform-d) δ 8.18 - 8.13 (m, 1H), 7.94 (d, J = 15.4 Hz, 1H), 7.86 (d, J = 7.8 Hz, 1H), 7.68 (dd, J = 7.9, 1.3 Hz, 1H), 7.58 (t, J = 7.7 Hz, 1H), 7.48 (t, J = 7.6 Hz, 1H), 7.43 - 7.32 (m, 5H), 5.32 (s, 1H), 1.40 - 1.33 (m, 1H), 1.21 - 1.13 (m, 1H), 0.98 - 0.89 (m, 1H), 0.48 (d, J = 345.4 Hz, 1H). HRMS (ESI): 388.1156 [M+H]⁺.

### Example 113 (S, E) -7-phenyl-6- (3- (2- (trifluoromethyl) phenyl) acryloyl) -4-oxa-6-azaspiro [2.4] heptan-5-one

Replace (E) -3- (o-methylphenyl) acrylic acid with (E) -3- (o-trifluoromethylphenyl) acrylic acid, and replace oxazolin-2-one with (S) -7-phenyl-4-oxa-6-azaspiro [2.4] heptan-5-one. The remaining required raw materials, reagents, and preparation methods were the same as in Example 1 to obtain (S, E) -7-phenyl-6- (3- (2- (trifluoromethylphenyl) phenyl) acryloyl) -4-oxa-6-azaspiro [2.4] heptan-5-one (yield 47.2%). ¹H NMR (500 MHz, Chloroform-d) δ 8.19 (ddd, J = 7.4, 1.6, 0.6 Hz, 1H), 8.02 (td, J = 7.4, 1.6 Hz, 1H), 7.96 (dd, J = 7.5, 1.6 Hz, 1H), 7.90 (td, J = 7.5, 1.6 Hz, 1H), 7.88 - 7.82 (m, 1H), 7.62 (d, J = 15.0 Hz, 1H), 7.37 - 7.24 (m, 5H), 5.20 (d, J = 0.7 Hz, 1H), 1.66 - 1.55 (m, 2H), 1.55 - 1.44 (m, 2H). LRMS (ESI): 388.12 [M+H]+.

### Example 114 (E) -5-phenyl-1- (3- (2- (trifluoromethyl) phenyl) acryloyl) imidazolidine-2,4-dione

Replace (E) -3- (o-methylphenyl) acrylic acid with (E) -3- (m-methoxyphenyl), and replace oxazolin-2-one with 5-phenylimidazolidine-2,4-dione. The remaining required raw materials, reagents, and preparation methods were the same as in Example 1 to obtain (E) -5-phenyl-1- (3-(2- (trifluoromethyl) phenyl) acryloyl) imidazolidine-2,4-dione (yield 62.8%). ¹H NMR (400 MHz, DMSO-d₆) δ 11.95 (s, 1H), 7.99 - 7.92 (m, 2H), 7.91 - 7.84 (m, 1H), 7.82 - 7.76 (m, 2H), 7.67 - 7.62 (m, 1H), 7.41 (d, J = 4.3 Hz, 4H), 7.39 - 7.34 (m, 1H), 5.67 (s, 1H). HRMS (ESI): 375.0952 [M+H]+.

### Example 115 (R, E) -6- (3- (3-methoxyphenyl) acryloyl) -7-phenyl-4-oxa-6-azaspiro [2.4] heptan-5-one

Replace (E) -3- (o-methoxyphenyl) acrylic acid with (E) -3- (methoxyphenyl) acrylic acid, and replace oxazolin-2-one with (R) -7-phenyl-4-oxa-6-azaspiro [2.4] heptan-5-one. The remaining required raw materials, reagents, and preparation methods were the same as in Example 1 to obtain (R, E) -6- (3- (3-methoxyphenyl) acryloyl) -7-phenyl-4-oxa-6-azaspiro [2.4] heptan-5-one (yield 43.2%). ¹H NMR (500 MHz, Chloroform-d) δ 7.93 (d, J = 15.7 Hz, 1H), 7.75 (d, J = 15.8 Hz, 2H), 7.40 - 7.25 (m, 8H), 7.19 (dt, J = 7.8, 1.4 Hz, 1H), 7.09 (dd, J = 2.6, 1.6 Hz, 1H), 6.97 - 6.91 (m, 2H), 5.32 (s, 1H), 1.41 - 1.32 (m, 1H), 1.20 - 1.10 (m, 1H), 0.96 - 0.87 (m, 1H), 0.51 - 0.42 (m, 1H). HRMS (ESI): 350.1384 [M+H]⁺.

### Example 116 (S, E) -6- (3- (3-methoxyphenyl) acryloyl) -7-phenyl-4-oxa-6-azaspiro [2.4] heptan-5-one

Replace (E) -3- (o-methoxyphenyl) acrylic acid with (E) -3- (methoxyphenyl) acrylic acid, and replace oxazolin-2-one with (S) -7-phenyl-4-oxa-6-azaspiro [2.4] heptan-5-one. The remaining required raw materials, reagents, and preparation methods were the same as in Example 1 to obtain (S, E) -6- (3- (3-methoxyphenyl) acryloyl) -7-phenyl-4-oxa-6-azaspiro [2.4] heptan-5-one (yield 48.2%).¹H NMR (500 MHz, Chloroform-d) δ 7.77 (d, J = 15.0 Hz, 1H), 7.72 - 7.65 (m, 1H), 7.37 - 7.23 (m, 7H), 7.15 (t, J = 1.5 Hz, 1H), 7.06 (ddt, J = 7.5, 3.1, 1.6 Hz, 2H), 5.20 (d, J = 0.7 Hz, 1H), 3.80 (s, 2H), 1.66 - 1.55 (m, 2H), 1.55 - 1.44 (m, 2H).LRMS (ESI): 350.15 [M+H]⁺.

### Example 117 (R, E) -6- (3- (3-phenoxyphenyl) acryloyl) -7-phenyl-4-oxa-6-azaspiro [2.4] heptan-5-one

Replace (E) -3- (o-methoxyphenyl) acrylic acid with (E) -3- (phenoxyphenyl) acrylic acid, and replace oxazolin-2-one with (R) -7-phenyl-4-oxa-6-azaspiro [2.4] heptan-5-one. The remaining required raw materials, reagents, and preparation methods were the same as in Example 1 to obtain (R, E) -6- (3- (3-phenoxyphenyl) acryloyl) -7-phenyl-4-oxa-6-azaspiro [2.4] heptan-5-one (yield 51.1%). ¹H NMR (500 MHz, Chloroform-d) δ 7.91 (d, J = 15.7 Hz, 1H), 7.72 (d, J = 15.7 Hz, 1H), 7.42 - 7.30 (m, 9H), 7.24 - 7.21 (m, 1H), 7.13 (t, J = 7.4 Hz, 1H), 7.04 - 6.99 (m, 3H), 5.31 (s, 1H), 1.39 - 1.32 (m, 1H), 1.19 - 1.11 (m, 1H), 0.96 - 0.88 (m, 1H), 0.52 - 0.42 (m, 1H). HRMS (ESI): 421.1542 [M+H]+.

### Example 118 (S, E) -6- (3- (3-phenoxyphenyl) acryloyl) -7-phenyl-4-oxa-6-azaspiro [2.4] heptan-5-one

Replace (E) -3- (o-methoxyphenyl) acrylic acid with (E) -3- (phenoxyphenyl) acrylic acid, and replace oxazolin-2-one with (S) -7-phenyl-4-oxa-6-azaspiro [2.4] heptan-5-one. The remaining required raw materials, reagents, and preparation methods were the same as in Example 1 to obtain (S, E) -6- (3- (3-phenoxyphenyl) acryloyl) -7-phenyl-4-oxa-6-azaspiro [2.4] heptan-5-one (yield 48.1%). ¹H NMR (500 MHz, Chloroform-d) δ 7.77 (d, J = 15.0 Hz, 1H), 7.72 - 7.65 (m, 1H), 7.40 - 7.24 (m, 10H), 7.20 - 7.15 (m, 1H), 7.18 - 7.00 (m, 6H), 5.19 (s, 1H), 1.66 - 1.44 (m, 5H). LRMS (ESI): 421.10 [M+H]⁺.

### Example 119 (R, E) -7-phenyl-6- (3- (2- (trifluoromethoxy) phenyl) acryloyl) -4-oxa-6-azaspiro [2.4] heptan-5-one

Replace (E) -3- (o-methylphenyl) acrylic acid with (E) -3- (trifluoromethoxyphenyl) acrylic acid, and replace oxazolin-2-one with (R) -7-phenyl-4-oxa-6-azaspiro [2.4] heptan-5-one. The remaining required raw materials, reagents, and preparation methods were the same as in Example 1 to obtain (R, E) -7-phenyl-6- (3- (2- (trifluoromethoxy) phenyl) acryloyl) -4-oxa-6-azaspiro [2.4] heptan-5-one (yield 34.2%).¹H NMR (500 MHz, Chloroform-d) δ 8.08 - 7.96 (m, 2H), 7.80 (dd, J = 7.8, 1.7 Hz, 1H), 7.45 - 7.24 (m, 8H), 5.32 (s, 1H), 1.41 - 1.32 (m, 1H), 1.19 - 1.12 (m, 1H), 0.97 = 0.90 (m, 1H), 0.51 - 0.43 (m, 1H). HRMS (ESI): 404.1105 [M+H]⁺.

### Example 120 (S, E) -7-phenyl-6- (3- (2- (trifluoromethoxy) phenyl) acryloyl) -4-oxa-6-azaspiro [2.4] heptan-5-one

Replace (E) -3- (o-methylphenyl) acrylic acid with (E) -3- (trifluoromethoxyphenyl) acrylic acid, and replace oxazolin-2-one with (S) -7-phenyl-4-oxa-6-azaspiro [2.4] heptan-5-one. The remaining required raw materials, reagents, and preparation methods were the same as in Example 1 to obtain (S, E) -7-phenyl-6- (3- (2- (trifluoromethoxy) phenyl) acryloyl) -4-oxa-6-azaspiro [2.4] heptan-5-one (yield 36.5%). ¹H NMR (500 MHz, Chloroform-d) δ 7.81 - 7.75 (m, 1H), 7.63 (d, J = 15.2 Hz, 1H), 7.56 (ddd, J = 7.5, 1.5, 0.6 Hz, 1H), 7.38 - 7.26 (m, 6H), 7.23 (dd, J = 7.5, 1.6 Hz, 1H), 6.99 (td, J = 7.5, 1.6 Hz, 1H), 5.19 (s, 1H), 1.66 - 1.55 (m, 2H), 1.55 - 1.44 (m, 2H). LRMS (ESI): 404.13 [M+H]⁺.

### Example 121 (R, E) -6- (3- (4-methoxyphenyl) acryloyl) -7-phenyl-4-oxa-6-azaspiro [2.4] heptan-5-one

Replace (E) -3- (o-methoxyphenyl) acrylic acid with (E) -4- (methoxyphenyl) acrylic acid, and replace oxazolin-2-one with (R) -7-phenyl-4-oxa-6-azaspiro [2.4] heptan-5-one. The remaining required raw materials, reagents, and preparation methods were the same as in Example 1 to obtain (R, E) -6- (3- (4-methoxyphenyl) acryloyl) -7-phenyl-4-oxa-6-azaspiro [2.4] heptan-5-one (yield 41.9%). ¹H NMR (600 MHz, Chloroform-d) δ 7.85 - 7.73 (m, 2H), 7.54 (d, J = 8.5 Hz, 2H), 7.40 - 7.30 (m, 5H), 6.89 (d, J = 8.6 Hz, 2H), 5.31 (s, 1H), 3.83 (s, 3H), 1.39 - 1.31 (m, 1H), 1.17 - 1.09 (m, 1H), 0.95 - 0.86 (m, 1H), 0.50 - 0.43 (m, 1H). LRMS (ESI): 350.40 [M+H]⁺.

### Example 122 (S, E) -6- (3- (4-methoxyphenyl) acryloyl) -7-phenyl-4-oxa-6-azaspiro [2.4] heptan-5-one

Replace (E) -3- (o-methoxyphenyl) acrylic acid with (E) -4- (methoxyphenyl) acrylic acid, and replace oxazolin-2-one with (S) -7-phenyl-4-oxa-6-azaspiro [2.4] heptan-5-one. The remaining required raw materials, reagents, and preparation methods were the same as in Example 1 to obtain (S, E) -6- (3- (4-methoxyphenyl) acryloyl) -7-phenyl-4-oxa-6-azaspiro [2.4] heptan-5-one (yield 41.9%). ¹H NMR (600 MHz, Chloroform-d) δ 7.85 - 7.73 (m, 2H), 7.54 (d, J = 8.5 Hz, 2H), 7.40 - 7.30 (m, 5H), 6.89 (d, J = 8.6 Hz, 2H), 5.31 (s, 1H), 3.83 (s, 3H), 1.39 - 1.31 (m, 1H), 1.17 - 1.09 (m, 1H), 0.95 - 0.86 (m, 1H), 0.50 - 0.43 (m, 1H). LRMS (ESI): 350.40 [M+H]⁺.

### Example 123 (R, E) -6- (3- ([1,1 '- biphenyl] -4-yl) acryloyl) -7-phenyl-4-oxa-6-azaspiro [2.4] heptan-5-one

Replace (E) -3- (o-methylphenyl) acrylic acid with (E) -3- ([1,1 '- biphenyl] -4-yl) acrylic acid, and replace oxazolin-2-one with (R) -7-phenyl-4-oxa-6-azaspiro [2.4] heptan-5-one. The remaining required raw materials, reagents, and preparation methods were the same as in Example 1 to obtain (R, E) -6- (3- ([1,1' - biphenyl] -4-yl) acryloyl) -7-phenyl-4-oxa-6-azaspiro [2.4] heptan-5-one (yield 33.2%). ¹H NMR (500 MHz, Chloroform-d) δ 7.97 (d, J = 15.7 Hz, 1H), 7.80 (d, J = 15.7 Hz, 1H), 7.68 - 7.56 (m, 6H), 7.47 - 7.28 (m, 8H), 5.30 (s, 1H), 1.39 - 1.31 (m, 1H), 1.18 - 1.09 (m, 1H), 0.96 - 0.87 (m, 1H), 0.50 - 0.42 (m, 1H). HRMS (ESI): 396.1599 [M+H]⁺.

### Example 124 (S, E) -6- (3- ([1,1 '- biphenyl] -4-yl) acryloyl) -7-phenyl-4-oxa-6-azaspiro [2.4] heptan-5-one

Replace (E) -3- (o-methylphenyl) acrylic acid with (E) -3- ([1,1 '- biphenyl] -4-yl) acrylic acid, and replace oxazolin-2-one with (S) -7-phenyl-4-oxa-6-azaspiro [2.4] heptan-5-one. The remaining required raw materials, reagents, and preparation methods were the same as in Example 1 to obtain (S, E) -6- (3- ([1,1' - biphenyl] -4-yl) acryloyl) -7-phenyl-4-oxa-6-azaspiro [2.4] heptan-5-one (yield 33.2%). ¹H NMR (500 MHz, Chloroform-d) δ 7.97 (d, J = 15.7 Hz, 1H), 7.80 (d, J = 15.7 Hz, 1H), 7.68 - 7.56 (m, 6H), 7.47 - 7.28 (m, 8H), 5.30 (s, 1H), 1.39 - 1.31 (m, 1H), 1.18 - 1.09 (m, 1H), 0.96 - 0.87 (m, 1H), 0.50 - 0.42 (m, 1H).LRMS (ESI): 396.16 [M+H]⁺.

### Example 125 (S, E) -3- (3- (4-methoxyphenyl) acryloyl) -4-phenyloxazolidin-2-one

Replace (E) -3- (o-methylphenyl) acrylic acid with (E) -4- (methoxyphenyl) acrylic acid, and replace oxazolidin-2-one with (R) -4-phenyloxazolidin-2-one. The remaining required raw materials, reagents, and preparation methods were the same as in Example 1 to obtain (S, E) -3-(3- (4-methoxyphenyl) acryloyl) -4-phenyloxazolidin-2-one (yield 48.2%). ¹H NMR (600 MHz, Chloroform-d) δ 7.86 - 7.76 (m, 2H), 7.59 - 7.56 (m, 2H), 7.43 - 7.40 (m, 2H), 7.39 - 7.34 (m, 3H), 6.94 - 6.90 (m, 2H), 5.58 (dd, J = 8.7, 3.9 Hz, 1H), 4.75 (t, J = 8.8 Hz, 1H), 4.32 (dd, J = 8.8, 3.9 Hz, 1H), 3.86 (s, 3H). HRMS (ESI): 324.1232 [M+H]⁺.

### Example 126 (S, E) -3- (3- (3-methoxyphenyl) acryloyl) -4-phenyloxazolidin-2-one

Replace (E) -3- (o-methylphenyl) acrylic acid with (E) -3- (methoxyphenyl) acrylic acid, and replace oxazolidin-2-one with (R) -4-phenyloxazolidin-2-one. The remaining required raw materials, reagents, and preparation methods were the same as in Example 1 to obtain (S, E) -3-(3- (3-methoxyphenyl) acryloyl) -4-phenyloxazolidin-2-one (yield 46.2%). ¹H NMR (600 MHz, Chloroform-d) δ 7.95 (d, J = 15.7 Hz, 1H), 7.77 (d, J = 15.7 Hz, 1H), 7.46 - 7.29 (m, 6H), 7.21 (d, J = 7.6 Hz, 1H), 7.12 (s, 1H), 6.96 (d, J = 8.2 Hz, 1H), 5.56 (d, J = 8.5 Hz, 1H), 4.73 (t, J = 8.8 Hz, 1H), 4.31 (d, J = 8.8 Hz, 1H), 3.84 (s, 3H). HRMS (ESI): 324.1233 [M+H]⁺.

### Example of Pharmacological Activity Test

### Example 1: Activity determination of the compound of the present invention for improving blood flow

We used a laser speckle test blood flow model to test the activity of compounds in improving cerebral blood flow in mice. The principle is to measure the intensity changes of flowing blood through laser speckle technology, reflecting real-time cerebral blood flow; By comparing the changes in blood flow before and after administration in mice, the effect of compounds on improving blood flow can be detected.

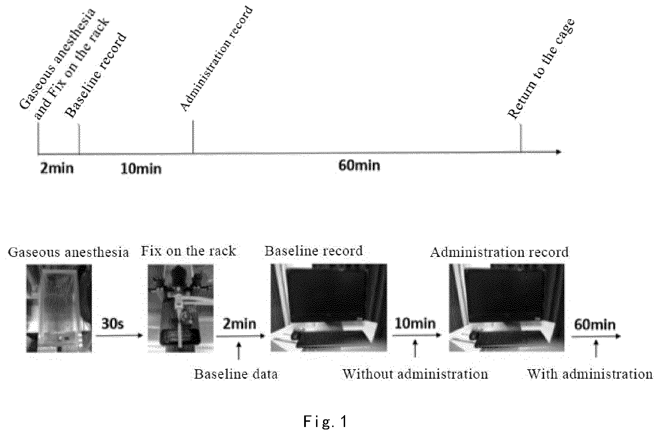

The experimental process is shown in Figure 1. The experimental mice were anesthetized for 30 seconds and fixed on a rack. After waiting for 2 minutes for the baseline light intensity data to stabilize, the brain blood flow light intensity data of the mice were recorded for 10 minutes without administration. Subsequently, the test compound was injected intraperitoneally and the cerebral blood flow and light intensity data of mice were recorded within 60 minutes after administration. Comparing the changes in light intensity before and after administration can reveal the degree of changes in cerebral blood flow in mice.

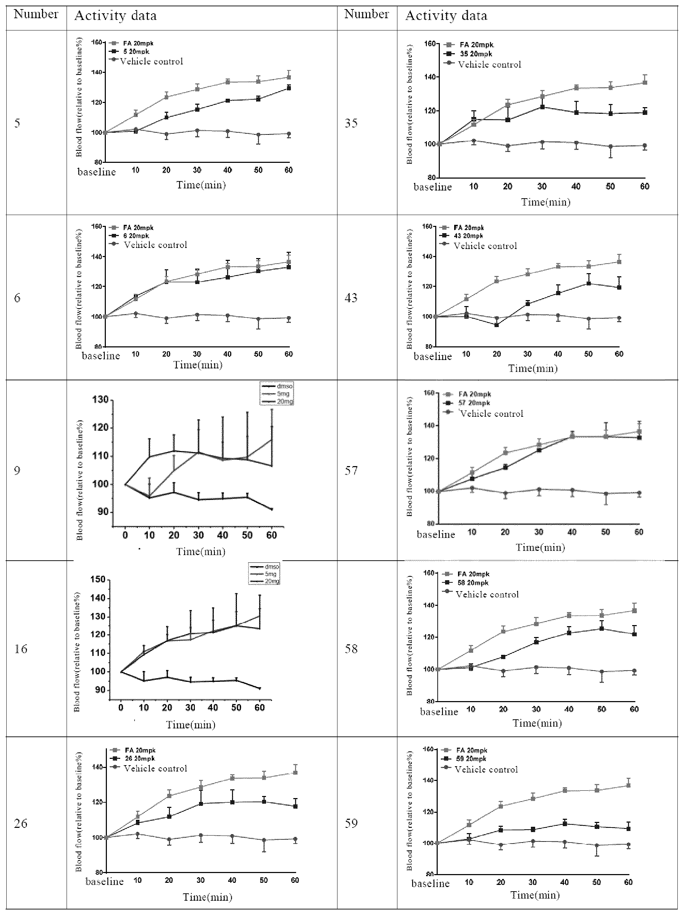

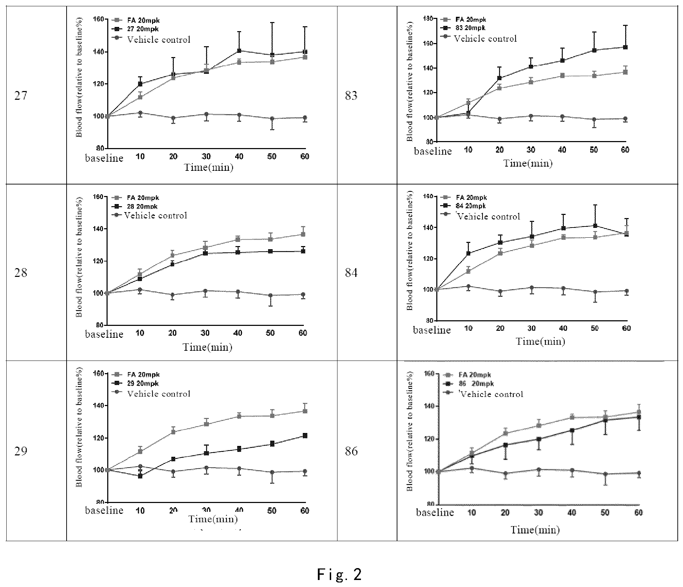

Through the laser speckle model mentioned above, we conducted preliminary tests on the activity of the compound in improving cerebral blood flow in mice. In this experiment, ferulic acid (FA) was used as a positive control. The blood flow improvement effect of the compound is shown in Figure 2. Multiple compounds have shown significant improvement effects on cerebral blood flow in this model. At a dose of 5 mg/kg, compound 9 can increase cerebral blood flow in mice by about 10%, and compound 16 can increase it by about 20%. At a dose of 20 mg/kg, compounds 30 and 59 increased mouse cerebral blood flow by about 10%, compounds 5, 6, 26, 28, 29, 35, 43, and 58 all increased mouse cerebral blood flow by about 20%, compounds 16, 57, and 86 increased mouse cerebral blood flow by about 30%, compounds 27, 84, and 104 increased it by about 40%, and compound 83 increased mouse cerebral blood flow by 50% to 60%. Overall, the patented compound has good development prospects.

### Example 2 Determination of pharmacokinetic parameters in the compound of the present invention

Male ICR (CD-1) mice were divided into two groups, with three mice in each group. Each group was orally administered 20mg/kg of the test substance or intravenously injected with 5mg/kg of the test substance, blood was collected before administration, and 0.25 h, 0.5 h, 1 h, 2 h, 4h, 8h, and 24 hours after administration, respectively, and centrifuged immediately to obtain plasma, and the drug concentration in the plasma was determined using liquid chromatography tandem mass spectrometry.

The pharmacokinetic parameters in mice were shown in the following table. In mice, the oral bioavailability of compounds 27 and 83 was 14.7% and 17.4%, respectively, indicating good pharmacokinetic properties.

**Table 2. Pharmacokinetic parameters of compounds 27 and 83 in mice**

| No. | Drug administration | Tₘₐₓ (h) | Cₘₐₓ (ng/mL) | AUC₀₋ₜ (ng·h/mL) | AUC_{0-∞} (ng·h/mL) | MRT_{0-∞} (h) | t_{1/2} (h) | CL (mL/min/kg) | F (%) |
|---|---|---|---|---|---|---|---|---|---|
| 27 | po-20mg/kg | 1 | 104 | 465 | 650 | 6.12 | 4.01 | / | 14.7 |
| | iv-5mg/kg | / | / | 790 | 805 | 1.04 | 2.08 | 104 | / |
| 83 | po-10mg/kg | 1 | 116 | 757 | 767 | 5.61 | 4.08 | / | 17.4 |
| | iv-5mg/kg | / | / | 2170 | 2200 | 1.21 | 1.56 | 37.8 | / |

In addition, by measuring the drug of the compound in brain tissue, it was found that the compound has good blood-brain barrier permeability, as shown in Table 3. Compound 27 has good brain tissue drug distribution after oral administration in mice, with a B/P of approximately 0.7.

**Table 3. Determination of drug concentration in brain tissue**

| Dose | Animal No. | Time (h) | Heart Plasma (ng/mL) | Portal Vein Plasma (ng/mL) | Brain (ng/g) | CSF (ng/mL) | Brain/H. Plasma |
|---|---|---|---|---|---|---|---|
| | 13 | **15** min | 41.9 | 320 | 29.4 | 1.63 | 0.7 |
| | 14 | | 117 | 375 | 75.4 | 8.37 | 0.65 |
| | 15 | | 52.7 | 418 | 47.9 | 1.40 | 0.91 |
| PO 20mg/kg | **mean** | | **70.4** | **371.1** | **50.9** | **3.8** | **0.7** |
| | 16 | **30 min** | 34.2 | 252 | 19.4 | 0.670 | 0.56 |
| | 17 | | 28.2 | 171 | 38.8 | 0.770 | 1.37 |
| | 18 | | 89.5 | 704 | 44.3 | 5.94 | 0.49 |
| | **mean** | | **61.9** | **373.1** | **43.7** | **3.2** | **0.7** |

All references mentioned in the present invention are cited as references in this application, as if each reference were cited separately. In addition, it should be understood that after reading the above teaching content of the present invention, those skilled in the art can make various changes or modifications to the present invention, and these equivalent forms also fall within the scope of the claims attached to this application.

## Claims

1. An α,β-unsaturated amide compound of formula I, or a racemic, a R-isomer, a S-isomer, a pharmaceutically acceptable salt, or a mixture thereof: 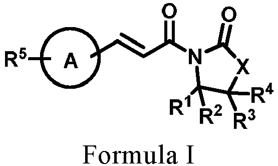 wherein,
R¹, R², R³, and R⁴ can each be independently selected from the group consisting of hydrogen, deuterium, tritium, halogen, cyano, amino, hydroxyl, nitro, substituted or unsubstituted C1~C6 alkyl, substituted or unsubstituted C1~C6 alkoxy and (CHR⁶)ₙR; wherein R is selected from the group consisting of: substituted or unsubstituted C6-C10 aryl, substituted or unsubstituted 5-7 membered heteroaryl, substituted or unsubstituted 5-7 membered heterocyclyl, substituted or unsubstituted C3-C12 cycloalkyl or fused heterocycle; or R³ and R⁴ together with the attached carbon atom form a group selected from the group consisting of carbonyl, substituted or unsubstituted 3-8-membered cycloalkyl, or substituted or unsubstituted 4-8 membered heterocyclyl;
Ⓐring is selected from the group consisting of: C6-C10 aryl, 5-12 membered heteroaryl, 5-12 membered heterocyclyl, C3-C12cycloalkyl or fused heterocycle;
R⁵ is 1, 2, 3, 4 or 5 substituents located on Ⓐ ring selected from the group consisting of hydrogen, deuterium, tritium, halogen, cyano, amino, hydroxyl, nitro, substituted or unsubstituted C1-C6 alkyl, substituted or unsubstituted C1-C6 alkoxy, substituted or unsubstituted C6-C10 aryl, substituted or unsubstituted C6-C10 aryloxy, substituted or unsubstituted 5-7 membered heteroaryl, substituted or unsubstituted 5-7 membered heteroaryloxy, substituted or unsubstituted 5-7 membered heterocycle, and substituted or unsubstituted C3-C12 cycloalkyl;
or two adjacent R⁵ are connected end-to-end with the atoms on Ⓐ ring to form a substituted or unsubstituted 4-8 membered ring (i.e., forming a fused ring structure with the A ring);
or two R⁵ on the same atom of Ⓐ ring are connected end-to-end together with Ⓐ ring form a substituted or unsubstituted 3-8-membered ring (i.e., forming a spiro ring structure with ring A);
X is N(CH₂)ₙR⁶, O, or S;
n is 0, 1, 2, or 3;
R⁶ is independently selected from the group consisting of: hydrogen, halogen, cyano, amino, hydroxyl, nitro, substituted or unsubstituted C1-C6 alkyl, substituted or unsubstituted C1-C6 alkoxy, substituted or unsubstituted C6-C10 aryl, substituted or unsubstituted 5-12 membered heterocyclyl containing 1 to 3 heteroatoms selected from oxygen, sulfur, and nitrogen, substituted or unsubstituted C2-C10 acyl, substituted or unsubstituted C2-C10 ester group, substituted or unsubstituted C1-C6 amide group, SO₂R⁵ and -COR⁵;
wherein, unless otherwise specified, the heteroaromatic ring, heterocycle, or heterocyclyl are each independently contains 1 to 4 heteroatoms selected from oxygen, sulfur, and nitrogen; the aromatic or heteroaromatic ring includes a monocyclic, fused-ring, or fused ring, and the carbocycle or heterocycle includes a monocyclic, fused, spiro, or bridged ring;
the "substituted" refers to being substituted by one or more (preferably 1-3) substituents selected from the group consisting of halogen, cyano, nitro, amino, hydroxyl, hydroxymethyl, carboxyl, thiol, C1-C6 alkyl, halogen substituted C1-C6 alkyl, C1-C6 alkoxy, halogen substituted C1-C6 alkoxy, C1-C6 alkoxycarbonyl, C2-C6 alkenyl, C2-C6 alkynyl, C3-C8 cycloalkyl, C1-C6 alkylsulfonyl, substituted or unsubstituted C6-C10 aryl, substituted or unsubstituted 5-7 membered heteroaryl, and 3-12 membered heterocyclyl;
the halogen is F, Cl, Br or I.

2. The α,β-unsaturated amide compound according to claim 1, wherein Ⓐ ring is selected from the group consisting of C6~C10 aryl and C5~C12 heteroaryl.

3. The α,β-unsaturated amide compound according to claim 1, wherein R¹, R², R³ and R⁴ are each independently selected from the group consisting of hydrogen, deuterium, substituted or unsubstituted C1-C6 alkyl and (CHR⁶)ₙR; wherein R is selected from the group consisting of: substituted or unsubstituted C6-C10 aryl, and substituted or unsubstituted 5-7 membered heteroaryl; n is 0, 1, or 2; R⁶ is hydrogen, halogen, or substituted or unsubstituted C1-C6 alkyl.

4. The α,β-unsaturated amide compound according to claim 1 or 2,wherein R¹, R², R³, and R⁴ are each independently selected from the group consisting of hydrogen, deuterium, substituted or unsubstituted C1-C6 alkyl and (CHR⁶)ₙR; wherein, R is selected from the group consisting of: substituted or unsubstituted C6-C10 aryl and substituted or unsubstituted 5-7 membered heteroaryl.

5. The α,β-unsaturated amide compound according to claim 1 or 2, wherein R¹ is H or D, and R² is selected from the group consisting of hydrogen, deuterium, substituted or unsubstituted C1-C6 alkyl and (CHR⁶)ₙR; wherein R is selected from the group consisting of: substituted or unsubstituted C6-C10 aryl, substituted or unsubstituted 5-7 membered heteroaryl; the "substituted" refers to being substituted by one or more substituents selected from the group consisting of halogen, cyano, nitro, amino, hydroxyl, hydroxymethyl, carboxyl, thiol, C1-C6 alkyl, halogen substituted C1-C6 alkyl, C1-C6 alkoxy, halogen substituted C1-C6 alkoxy, and C1-C6 alkoxycarbonyl.

6. The α,β-unsaturated amide compound according to claim 1 or 2, wherein R³ and R⁴ are each independently deuterium.

7. The α,β-unsaturated amide compound according to claim 1 or 2, wherein the compound of formula I is selected from the following table:
| number | structure | name |
|---|---|---|
| 1 | 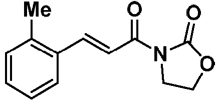 | (E) 3- (3- (o-methylphenyl) acryloyl) oxazolidin-2-one |
| 2 | 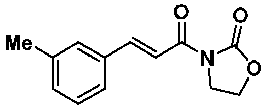 | (E) 3- (3- (m-methylphenyl) acryloyl) oxazolidin-2-one |
| 3 | 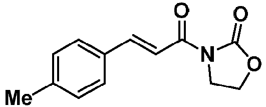 | (E) 3- (3- (p-methylphenyl) acryloyl) oxazolidin-2-one |
| 4 | 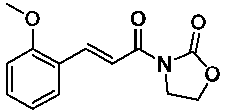 | (E) 3- (3- (2-methoxyphenyl) acryloyl) oxazolidin-2-one |
| 5 | 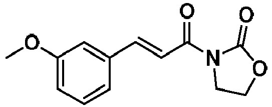 | (E) 3- (3- (3-methoxyphenyl) acryloyl) oxazolidin-2-one |
| 6 | 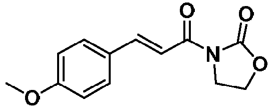 | (E) 3- (3- (4-methoxyphenyl) acryloyl) oxazolidin-2-one |
| 7 | 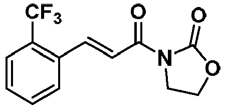 | (E) 3- (3- (2- (trifluoromethylphenyl) acryloyl) oxazolidin-2-one |
| 8 | 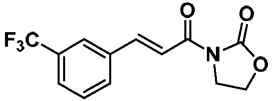 | (E) 3- (3- (3- (trifluoromethylphenyl) acryloyl) oxazolidin-2-one |
| 9 | 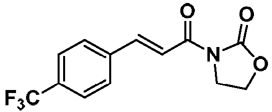 | (E) 3- (3- (4- (trifluoromethylphenyl) acryloyl) oxazolidin-2-one |
| 10 | 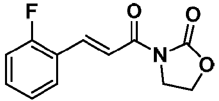 | (E) 3- (3- (2-fluorophenyl) acryloyl) oxazolidin-2-one |
| 11 | 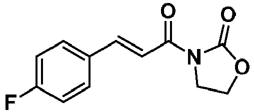 | (E) 3- (3- (4-fluorophenyl) acryloyl) oxazolidin-2-one |
| 12 | 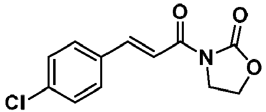 | (E) 3- (3- (4-chlorophenyl) acryloyl) oxazolidin-2-one |
| 13 | 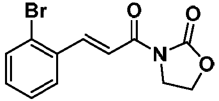 | (E) 3- (3- (2-bromophenyl) acryloyl) oxazolidin-2-one |
| 14 | 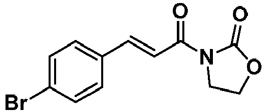 | (E) 3- (3- (4-bromophenyl) acryloyl) oxazolidin-2-one |
| 15 | 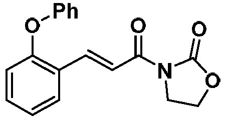 | (E) 3- (3- (2-phenoxyphenyl) acryloyl) oxazolidin-2-one |
| 16 | 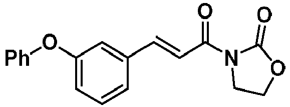 | (E) 3- (3- (3-phenoxyphenyl) acryloyl) oxazolidin-2-one |
| 17 | 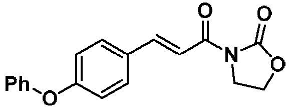 | (E) 3- (3- (4-phenoxyphenyl) acryloyl) oxazolidin-2-one |
| 18 | 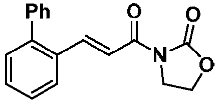 | (E) 3- (3- ([1,1'- biphenyl] -2-yl) acryloyl) oxazolidin-2-one |
| 19 | 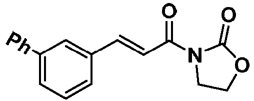 | (E) 3- (3- ([1,1'- biphenyl] -3-yl) acryloyl) oxazolidin-2-one |
| 20 | 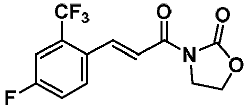 | (E) 3- (3- (4-fluoro-2-trifluoromethylphenyl) acryloyl) oxazolidin-2-one |
| 21 | 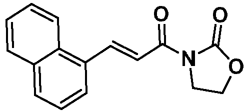 | (E) 3- (3- (naphthalen-1-yl) acryloyl) oxazolidin-2-one |
| 22 | 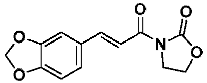 | (E) 3- (3- (benzo [d] [1,3] dioxolan-5-yl) acryloyl) oxazolidin-2-one |
| 23 | 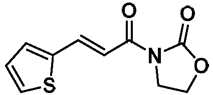 | (E) 3- (3- (thiphen-2-yl) acryloyl) oxazolidin-2-one |
| 24 | 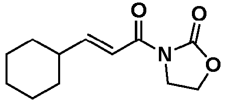 | (E) 3- (3-cyclohexylcacryloyl) oxazolidin-2-one |
| 26 | 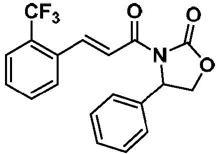 | (E) 3- (3- (2-trifluoromethylphenyl) acryloyl) -4-phenyloxazolidin-2-one |
| 27 | 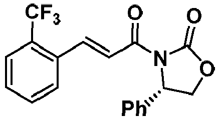 | (S, E) -3- (3- (2-trifluoromethylphenyl) acryloyl) -4-phenyloxazolidin-2-one |
| 28 | 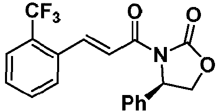 | (R, E) -3- (3- (2-trifluoromethylphenyl) acryloyl) -4-phenyloxazolidin-2-one |
| 29 | 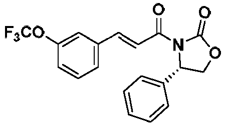 | (S, E) -4-phenyl-3- (3- (3-(trifluoromethoxy) phenyl) acryloyl) oxazolidin-2-one |
| 30 | 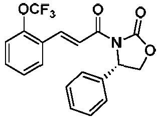 | (S, E) -4-phenyl-3- (3- (2-(trifluoromethoxy) phenyl) acryloyl) oxazolidin-2-one |
| 31 | 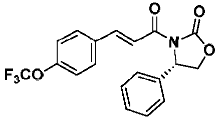 | (S, E) -4-phenyl-3- (3- (4-(trifluoromethoxy) phenyl) acryloyl) oxazolidin-2-one |
| 32 | 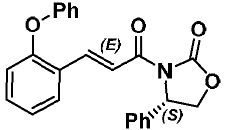 | (S, E) -3- (3- (2-phenoxyphenyl) acryloyl) -4-phenyloxazolidin-2-one |
| 33 | 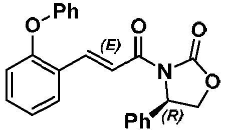 | (R, E) -3- (3- (2-phenoxyphenyl) acryloyl) -4-phenyloxazolidin-2-one |
| 34 | 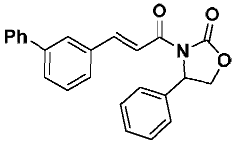 | (E) 3- (3- ([1,1 '- biphenyl] -3-yl) acryloyl) -4-phenyloxazolidin-2-one |
| 35 | 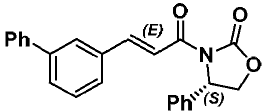 | (S, E) -3- (3- ([1,1 '- biphenyl] -3-yl) acryloyl) -4-phenyloxazolidin-2-one |
| 36 | 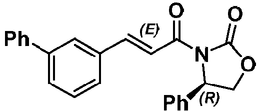 | (R, E) -3- (3- ([1,1 '- biphenyl] -3-yl) acryloyl) -4-phenyloxazolidin-2-one |
| 37 | 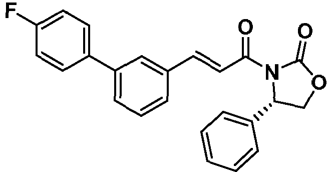 | (S, E) -3- (3- (4 '- fluorine - [1,1' - biphenyl] -3-yl) acryloyl) -4-phenyloxazolidin-2-one |
| 38 | 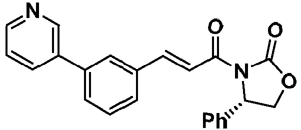 | (S, E) -4-phenyl-3- (3- (pyridin-3-yl) phenyl) acryloyl) oxazolidin-2-one |
| 39 | 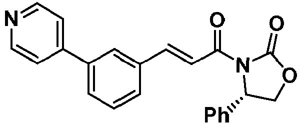 | (S, E) -4-phenyl-3- (3- (pyridin-4-yl) phenyl) acryloyl) oxazolidin-2-one |
| 40 | 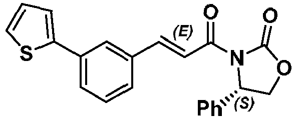 | (S, E) -4-phenyl-3- (3- (3- (thiphen-2-yl) phenyl) acryloyl) oxazolidin-2-one |
| 41 | 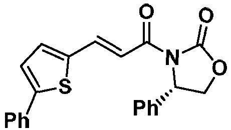 | (S, E) -4-phenyl-3- (3- (5-phenylthiophen-2-yl) acryloyl) oxazolidin-2-one |
| 42 | 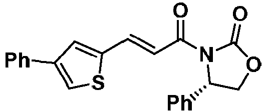 | (S, E) -4-phenyl-3- (3- (4-phenylthiophen-2-yl) acryloyl) oxazolidin-2-one |
| 43 | 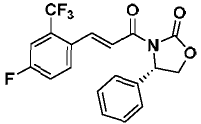 | (S, E) -3- (3- (4-fluoro-2-(trifluoromethyl) phenyl) acryloyl) -4-phenyloxazolidin-2-one |
| 44 | 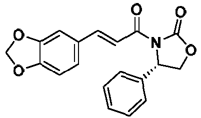 | (S, E) -3- (3- (benzo [d] [1,3] dioxolan-5-yl) acryloyl) -4-phenyloxazolidin-2-one |
| 45 | 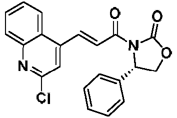 | (S, E) -3- (3- (2-chloroquinolin-4-yl) acryloyl) -4-phenyloxazolidin-2-one |
| 46 | 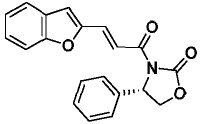 | (S, E) -3- (3- (benzofuran-2-yl) acryloyl) -4-phenyloxazolidin-2-one |
| 47 | 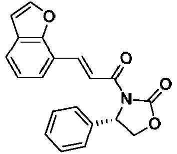 | (S, E) -3- (3- (benzofuran-7-yl) acryloyl) -4-phenyloxazolidin-2-one |
| 48 | 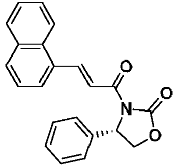 | (S, E) -3- (3- (naphthalen-1-yl) acryloyl) -4-phenyloxazolidin-2-one |
| 49 | 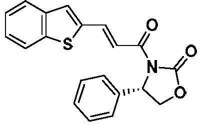 | (S, E) -3- (3- (benzo [b] thiophen-2-yl) acryloyl) -4-phenyloxazolidin-2-one |
| 50 | 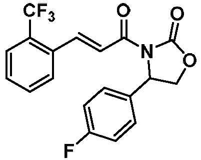 | (E) 4- (4 '- fluorophenyl) -3- (3- (2-(trifluoromethylphenyl) acryloyl) oxazolidin-2-one |
| 51 | 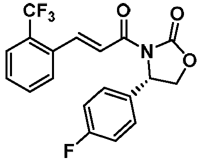 | (S, E) -4- (4 '- fluorophenyl) -3- (3- (2-(trifluoromethylphenyl) acryloyl) oxazolidin-2-one |
| 52 | 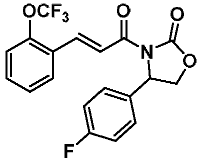 | (E) 4- (4 '- fluorophenyl) -3- (3- (2-(trifluoromethoxyphenyl) acryloyl) oxazolidin-2-one |
| 53 | 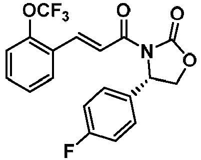 | (S, E) -4- (4 '- fluorophenyl) -3- (3- (2-(trifluoromethoxyphenyl) acryloyl) oxazolidin-2-one |
| 54 | 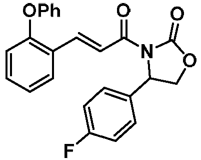 | (E) 4- (4 '- fluorophenyl) -3- (3- (2-phenoxyphenyl) acryloyl) oxazolidin-2-one |
| 55 | 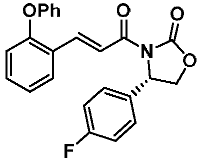 | (S, E) -4- (4 '- fluorophenyl) -3- (3- (2-phenoxyphenyl) acryloyl) oxazolidin-2-one |
| 56 | 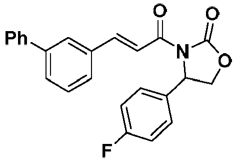 | (E) 3- (3- ([1,1 '- biphenyl] -3-yl) acryloyl) -4- (4-fluorophenyl) oxazolidin-2-one |
| 57 | 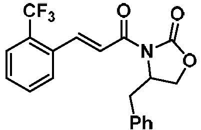 | (E) 4-benzyl-3- (3- (2-trifluoromethylphenyl) acryloyl) oxazolidin-2-one |
| 58 | 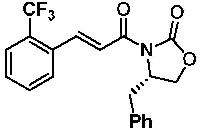 | (S, E) -4-benzyl-3- (3- (2-trifluoromethylphenyl) acryloyl) oxazolidin-2-one |
| 59 | 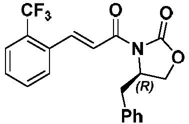 | (R, E) -4-benzyl-3- (3- (2-trifluoromethylphenyl) acryloyl) oxazolidin-2-one |
| 60 | 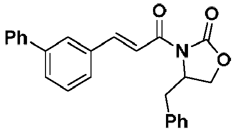 | (E) 3- (3- ([1,1 '- biphenyl] -3-yl) acryloyl) -4-benzyloxazolidin-2-one |
| 61 | 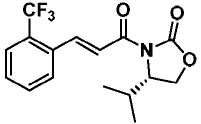 | (S, E) -4-isopropyl-3- (3- (2-(trifluoromethyl) phenyl) acryloyl) oxazolidin-2-one |
| 62 | 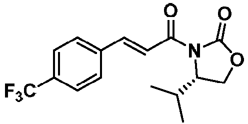 | (S, E) -4-isopropyl-3- (3- (4-(trifluoromethyl) phenyl) acryloyl) oxazolidin-2-one |
| 63 | 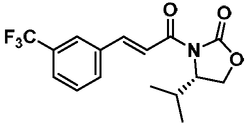 | (S, E) -4-isopropyl-3- (3- (3-(trifluoromethyl) phenyl) acryloyl) oxazolidin-2-one |
| 64 | 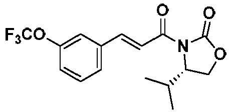 | (S, E) -4-isopropyl-3- (3- (3-trifluoromethoxyphenyl) acryloyl) oxazolidin-2-one |
| 65 | 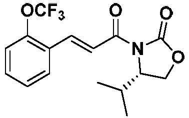 | (S, E) -4-isopropyl-3- (3- (2-trifluoromethoxyphenyl) acryloyl) oxazolidin-2-one |
| 66 | 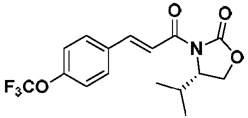 | (S, E) -4-isopropyl-3- (3- (4-trifluoromethoxyphenyl) acryloyl) oxazolidin-2-one |
| 67 | 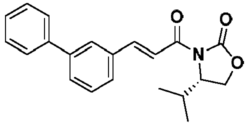 | (S, E) -3- (3- ([1,1 '- biphenyl] -3-yl) acryloyl) -4-isopropyloxazolidin-2-one |
| 68 | 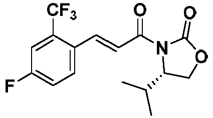 | (S, E) -3- (3- (4-fluoro-2-(trifluoromethyl) phenyl) acryloyl) -4-isopropyloxazolidin-2-one |
| 69 | 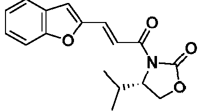 | (S, E) -3- (3- (benzofuran-2-yl) acryloyl) -4-isopropyloxazolidin-2-one |
| 70 | 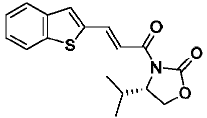 | (S, E) -3- (3- (benzo [b] thiophen-2-yl) acryloyl) -4-isopropyloxazolidin-2-one |
| 71 | 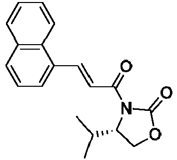 | (S, E) -4-isopropyl-3- (3- (naphthalen-1-yl) acryloyl) oxazolidin-2-one |
| 72 | 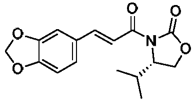 | (S, E) -3- (3- (benzo [d] [1,3] dioxolan-5-yl) acryloyl) -4-isopropyloxazolidin-2-one |
| 73 | 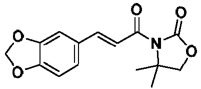 | (E) 3- (3- (benzo [d] [1,3] dioxolan-5-yl) acryloyl) -4,4-dimethyloxazolidin-2-one |
| 74 | 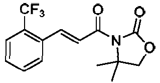 | (E)-4, 4-dimethyl-3- (3- (2-(trifluoromethyl) phenyl) acryloyl) oxazolidin-2-one |
| 75 | 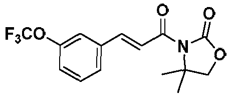 | (E)-4, 4-dimethyl-3- (3- (3-(trifluoromethoxy) phenyl) acryloyl) oxazolidin-2-one |
| 76 | 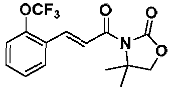 | (E)-4, 4-dimethyl-3- (3- (2-(trifluoromethoxy) phenyl) acryloyl) oxazolidin-2-one |
| 77 | 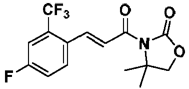 | (E) 3- (3- (4-fluoro-2- (trifluoromethyl) phenyl) acryloyl) -4,4-dimethyloxazolidin-2-one |
| 78 | 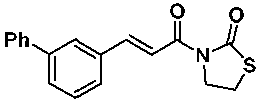 | (E) 3- (3- ([1,1 '- biphenyl] -3-yl) acryloyl) thiazolidin-2-one |
| 79 | 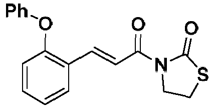 | (E) 3- (3- (2-phenoxyphenyl) acryloyl) thiazolidin-2-one |
| 80 | 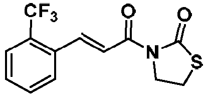 | (E) 3- (3- (2-trifluoromethylphenyl) acryloyl) thiazolidin-2-one |
| 81 | 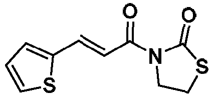 | (E) 3- (3- (thiophen-2-yl) acryloyl) thiazolidin-2-one |
| 83 | 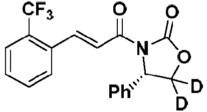 | (S, E) -4-phenyl-3- (3- (2-trifluoromethylphenyl) acryloyl) oxazolidin-2-one-5,5-d₂ |
| 84 | 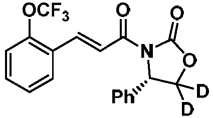 | (S, E) -4-phenyl-3- (3- (2-trifluoromethoxyphenyl) acryloyl) oxazolidin-2-one-5,5-d₂ |
| 85 | 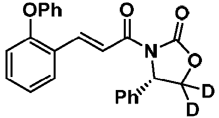 | (S, E) -4-phenyl-3- (3- (2-phenoxyphenyl) acryloyl) oxazolidin-2-one-5,5-*d₂* |
| 86 | 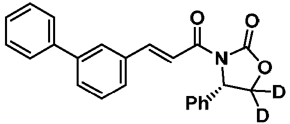 | (S, E) -3- (3- ([1,1 '- biphenyl] -3-yl) acryloyl) -4-phenyloxazolidin-2-one-5,5-d₂ |
| 87 | 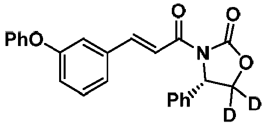 | (S, E) -4-phenyl-3- (3- (3-phenoxyphenyl) acryloyl) oxazolidin-2-one-5,5-*d₂* |
| 88 | 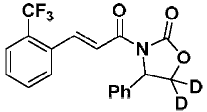 | (E) 4-phenyl-3- (3- (2-trifluoromethylphenyl) acryloyl) oxazolidin-2-one-5,5-d₂ |
| 89 | 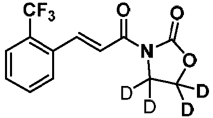 | (E) 3- (3- (2-trifluoromethylphenyl) acryloyl) oxazolidin-2-one-4,4,5-d4 |
| 90 | 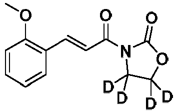 | (E) 3- (3- (2-methoxyphenyl) acryloyl) oxazolidin-2-one-4,4,5-d4 |
| 91 | 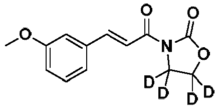 | (E) 3- (3- (3-methoxyphenyl) acryloyl) oxazolidin-2-one-4,4,5-d4 |
| 92 | 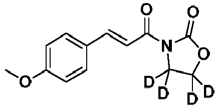 | (E) 3- (3- (4-methoxyphenyl) acryloyl) oxazolidin-2-one-4,4,5-d4 |
| 93 | 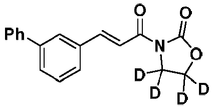 | (E) 3- (3- ([1,1 '- biphenyl] -3-yl) acryloyl) oxazolidin-2-one-4,4,5-d4 |
| 94 | 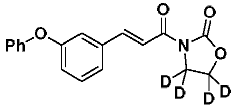 | (E) 3- (3- (3-phenoxyphenyl) acryloyl) oxazolidin-2-one-4,4,5-d4 |
| 95 | 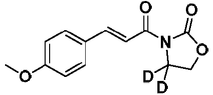 | (E) 3- (3- (4-methoxyphenyl) acryloyl) oxazolidin-2-one-4,4-d₂ |
| 96 | 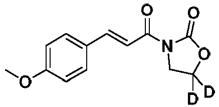 | (E) 3- (3- (4-methoxyphenyl) acryloyl) oxazolidin-2-one-5,5-d₂ |
| 97 | 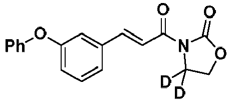 | (E) 3- (3- (3-phenoxyphenyl) acryloyl) oxazolidin-2-one-4,4-d₂ |
| 98 | 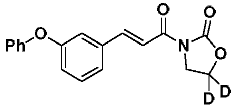 | (E) 3- (3- (3-phenoxyphenyl) acryloyl) oxazolidin-2-one-5,5-d₂ |
| 99 | 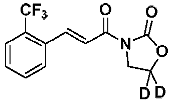 | (E) 3- (3- (2-trifluoromethylphenyl) acryloyl)oxazolidin-2-one-5,5-d₂ |
| 100 | 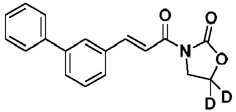 | (E) 3- (3- ([1,1 '- biphenyl] -3-yl) acryloyl) oxazolidin-2-one-5,5-d₂ |
| 101 | 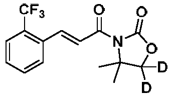 | (E)-4, 4-dimethyl-3- (3- (2-(trifluoromethyl) phenyl) acryloyl) oxazolidin-2-one-5,5-d₂ |
| 102 | 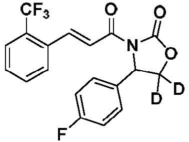 | (E) 4- (4 '- fluorophenyl) -3- (3- (2-(trifluoromethylphenyl) acryloyl) oxazolidin-2-one-5,5-d₂ |
| 103 | 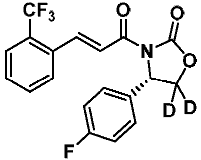 | (S, E) -4- (4 '- fluorophenyl) -3- (3- (2-(trifluoromethylphenyl) acryloyl) oxazolidin-2-one-5,5-d₂ |
| 104 | 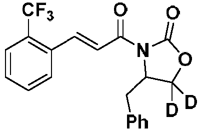 | (E) 4-Benzyl-3- (3- (2-trifluoromethylphenyl) acryloyl) oxazolidin-2-one-5,5-d₂ |
| 105 | 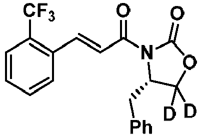 | (S, E) -4-benzyl-3- (3- (2-trifluoromethylphenyl) acryloyl) oxazolidin-2-one-5,5-d₂ |
| 106 | 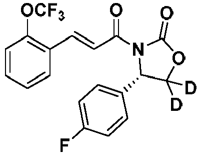 | (S, E) -4- (4 '- fluorophenyl) -3- (3- (2-(trifluoromethoxyphenyl) acryloyl) oxazolidin-2-one-5,5-d₂ |
| 107 | 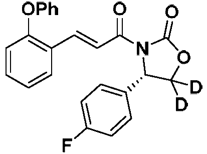 | (S, E) -4- (4 '- fluorophenyl) -3- (3- (2-(phenoxyphenyl) acryloyl) oxazolidin-2-one-5,5-d₂ |
| 108 | 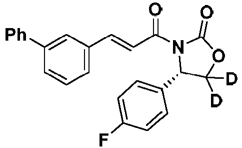 | (S, E) -3- (3- ([1,1 '- biphenyl] -3-yl) acryloyl) -4- (4' - fluorophenyl) oxazolidin-2-one-5,5-d₂ |
| 109 | 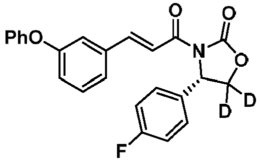 | (S, E) -4- (4 '- fluorophenyl) -3- (3- (3-(phenoxyphenyl) acryloyl) oxazolidin-2-one-5,5-d₂ |
| 110 | 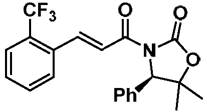 | (R,E)-5, 5-dimethyl-4-phenyl-3- (3- (2-(trifluoromethyl) phenyl) acryloyl) oxazolidin-2-one |
| 111 | 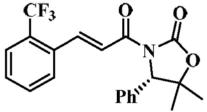 | (S,E)-5, 5-dimethyl-4-phenyl-3- (3- (2-(trifluoromethyl) phenyl) acryloyl) oxazolidin-2-one |
| 112 | 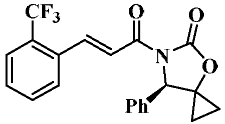 | (R, E) -7-phenyl-6- (3- (2-(trifluoromethyl) phenyl) acryloyl) -4-oxa-6-azaspiro [2.4] heptan-5-one |
| 113 | 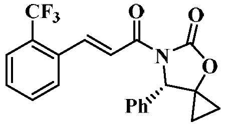 | (S, E) -7-phenyl-6- (3- (2-(trifluoromethyl) phenyl) acryloyl) -4-oxa-6-azaspiro [2.4] heptan-5-one |
| 114 | 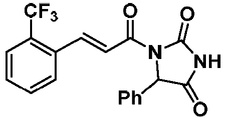 | (E) 5-phenyl-1- (3- (2- (trifluoromethyl) phenyl) acryloyl) imidazolidine-2,4-diketone |
| 115 | 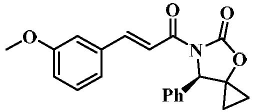 | (R, E) -6- (3- (3-methoxyphenyl) acryloyl) -7-phenyl-4-oxa-6-azaspiro [2.4] heptan-5-one |
| 116 | 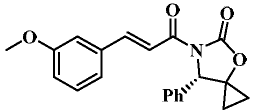 | (S, E) -6- (3- (3-methoxyphenyl) acryloyl) -7-phenyl-4-oxa-6-azaspiro [2.4] heptan-5-one |
| 117 | 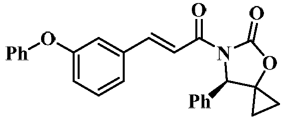 | (R, E) -6- (3- (3-phenoxyphenyl) acryloyl) -7-phenyl-4-oxa-6-azaspiro [2.4] heptan-5-one |
| 118 | 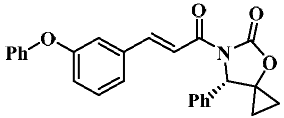 | (S, E) -6- (3- (3-phenoxyphenyl) acryloyl) -7-phenyl-4-oxa-6-azaspiro [2.4] heptan-5-one |
| 119 | 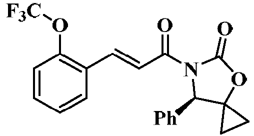 | (R, E) -7-phenyl-6- (3- (2-(trifluoromethoxy) phenyl) acryloyl) -4-oxa-6-azaspiro [2.4] heptan-5-one |
| 120 | 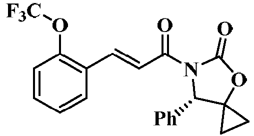 | (S, E) -7-phenyl-6- (3- (2-(trifluoromethoxy) phenyl) acryloyl) -4-oxa-6-azaspiro [2.4] heptan-5-one |
| 121 | 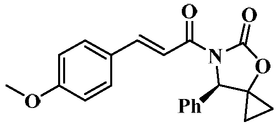 | (R, E) -6- (3- (4-methoxyphenyl) acryloyl) -7-phenyl-4-oxa-6-azaspiro [2.4] heptan-5-one |
| 122 | 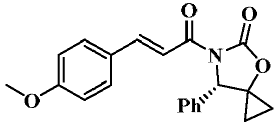 | (S, E) -6- (3- (4-methoxyphenyl) acryloyl) -7-phenyl-4-oxa-6-azaspiro [2.4] heptan-5-one |
| 123 | 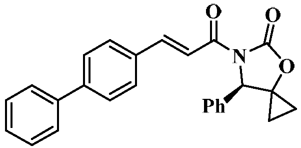 | (R, E) -6- (3- ([1,1 '- biphenyl] -4-yl) acryloyl) -7-phenyl-4-oxa-6-azaspiro [2.4] heptan-5-one |
| 124 | 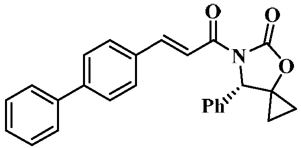 | (S, E) -6- (3- ([1,1 '- biphenyl] -4-yl) acryloyl) -7-phenyl-4-oxa-6-azaspiro [2.4] heptan-5-one |
| 125 | 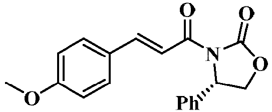 | (S, E) -3- (3- (4-methoxyphenyl) acryloyl) -4-phenyloxazolidin-2-one |
| 126 | 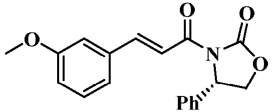 | (S, E) -3- (3- (3-methoxyphenyl) acryloyl) -4-phenyloxazolidin-2-one |

8. The preparation method of compound of formula I according to claim 1, wherein the method comprises steps of: 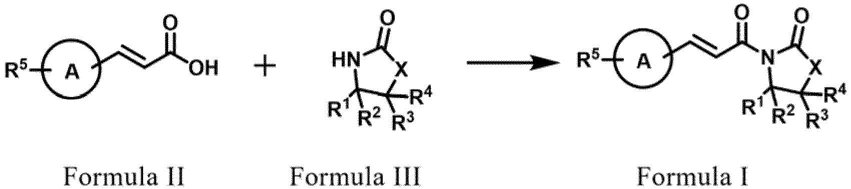 In an inert solvent, reacting a compound of formula II and a compound of formula III to obtain compound of formula I.

9. A pharmaceutical composition, comprising (1) a compound according to claim 1, or a stereoisomer, a tautomer, , a pharmaceutically acceptable salt, a hydrate or a solvate thereof; and (2) a pharmaceutically acceptable carrier.

10. A use of the compound according to claim 1, or a stereoisomer, a tautomer, a pharmaceutically acceptable salt, a hydrate or a solvate thereof, or the pharmaceutical composition as claimed in claim 9in the preparation of a pharmaceutical composition for the prevention and/or treatment of neurodegenerative diseases or stroke; preferably, the neurodegenerative disease is selected from the group consisting of Alzheimer's disease and vascular dementia.
